(19) Europäisches Patentamt
European Patent Office
Office européen des brevets

(11) Veröffentlichungsnummer: **0 153 278**
**B1**

(12) # EUROPÄISCHE PATENTSCHRIFT

(45) Veröffentlichungstag der Patentschrift: **08.06.88**

(51) Int. Cl.⁴: **C 07 D 487/22**, C 11 D 3/395, C 09 B 47/04, D 06 M 16/00, A 01 N 43/90

(21) Anmeldenummer: **85810051.4**

(22) Anmeldetag: **11.02.85**

(54) **Wasserlösliche Phthalocyaninverbindungen und deren Verwendung als Photoaktivatoren.**

(30) Priorität: **17.02.84 CH 779/84**

(43) Veröffentlichungstag der Anmeldung: **28.08.85 Patentblatt 85/35**

(45) Bekanntmachung des Hinweises auf die Patenterteilung: **08.06.88 Patentblatt 88/23**

(84) Benannte Vertragsstaaten: **CH DE FR GB IT LI**

(56) Entgegenhaltungen:
**EP-A-0 003 149**
**EP-A-0 003 371**
**EP-A-0 003 861**
**EP-A-0 054 992**
**DE-A-2 812 261**
**FR-A-1 029 638**
**FR-A-2 341 363**
**US-A-3 094 535**
**US-A-3 094 536**
**US-A-3 137 703**

**JOURNAL OF THE AMERICAN CHEMICAL SOCIETY, Band 82, Nr. 22, 23. November 1960, Seiten 5790-5791, Gaston, US; R.D. JOYNER u.a.: "Germanium phthalocyanines"**

(73) Patentinhaber: **CIBA- GEIGY AG, Klybeckstrasse 141, CH- 4002 Basel (CH)**

(72) Erfinder: **Graf, Gregor, Dr., Bruderholzrain 28, CH- 4059 Basel (CH)**
Erfinder: **Hölzle, Gerd, Dr., Spitzacker 9, CH- 4410 Liestal (CH)**
Erfinder: **Reinert, Gerhard, Dr., Weiherweg 1/7, CH- 4123 Allschwil (CH)**

**EP 0 153 278 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue wasserlösliche Phthalocyaninverbindungen, Verfahren zu deren Herstellung, deren Verwendung als Photoaktivatoren (Photosensibilisatoren) bzw. Singlett-Sauerstoff-Produzenten, insbesondere zum Bleichen von bzw. Fleckenentfernen aus Textilien und zum Bekämpfen von Mikroorganismen in oder auf organischen oder anorganischen Substraten, sowie Bleich-, Wasch-, Spül- und Einweichmittel bzw. antimikrobiell wirksame Mittel, die die neuen Phthalocyaninverbindungen enthalten.

Es ist bekannt, dass verschiedene wasserlösliche Phthalocyaninverbindungen, insbesondere solche mit Zink und Aluminium als Zentralatom, photosensibilisierende Wirkung zeigen und daher als Photobleichmittel bzw. antimikrobielle Wirkstoffe verwendet werden können. Siehe dazu, unter anderen, US-A-3 927 967, 4 033 718, 4 166 718 und 4 094 806; DE-A-2 222 829, 2 627 449 und 2 812 261; EP-A-3 149, 3 371, 3 861, 26 744, 35 470, 47 716, 54 992 und 81 462. Die genannten Publikationen beschreiben auch Mittel, die die genannten wasserlöslichen Phthalocyaninverbindungen enthalten.

Ferner werden in der US-3 094 535 und dem Journal of the American Chemical Society, Band 82, Nr. 22, 23 (1970) Seiten 5790-5791 wasserunlösliche Germanium - Phthalocyaninverbindungen beschrieben.

Die neuen wasserlöslichen Phthalocyaninverbindungen gemäss vorliegender Erfindung enthalten als Zentralatom Ge(IV). Sie entsprechen der allgemeinen Formel

$$\left[\mathrm{MePc}\right]\begin{matrix}(AZ)_n\\ (Y)_p\end{matrix} \quad , (1)$$

worin MePc für das Phthalocyanin- oder Napthalocyaninringsystem steht, A eine anionische, nichtionische oder kationische wasserlöslichmachende Gruppe,

Z das Gegenion zu A, falls A eine ionische, und Z ist Null, falls A eine nichtionische Gruppe ist,

n eine beliebige Zahl von 1 bis 8,

Y einen neutralen, nicht wasserlöslichmachenden Substituenten und

p eine beliebige Zahl von 0 bis 6 bedeuten, wobei die im Molekül vorhandenen Gruppen AZ und Y jeweils gleich oder verschieden sein können, und die Summe n + p höchstens 8 beträgt.

Wie aus der Phthalocyaninchemie hinreichend bekannt, sind die freien Valenzen der Zentralatome je nach Wertigkeit mit einem oder mehreren zusätzlichen Liganden, z. B. mit Anionen, abgesättigt. Diese Anionen können z. B. mit den Anionen der jeweiligen Verbindung identisch sein, die zur Herstellung des Komplexes benutzt wurde. Beispiele für solche Anionen sind etwa Halogenid-, Sulfat-, Nitrat-, Phosphat-, Hydroxylionen, Ionen von organischen Carbonsäuren (z. B. Acetat-, Formiation) oder Sulfonsäuren (z. B. Tosylation).

Aus der Phthalocyaninchemie ist ebenfalls bekannt, dass die Phthalo cyaninverbindungen häufig keine einheitlichen Substanzen sind, sondern oft Gemische darstellen. Daher ist die im Molekül vorhandene Anzahl der Substituenten (Substitutionsgrad) selten eine ganze Zahl (n, p nicht ganzzahlig). Ebenso ist festzuhalten, dass die einzelnen Substituenten A bzw. Y jeweils gleich oder verschieden sein können, d.h., dass in einem Molekül auch durchaus verschiedene wasserlöslichmachende bzw. neutrale Substituenten vorhanden sein können. Die wasserlöslichmachenden Substituenten können dabei entweder vom gleichen Typus sein (kationisch, anionisch oder nichtionisch) oder sie können verschiedenen Typen angehören, wobei alle Kombinationen (anionisch/nichtionisch, kationisch/nichtionisch, anionisch/kationisch und anionisch/nichtionisch/kationisch) möglich sind. Alle Substituenten A und Y sind an die Phenylringe des Phthalocyanin- bzw. Naphthalocyaninringsystems gebunden.

Unter "Naphthalocyaninringsystem" ist ein Phthalocyaninringsystem zu verstehen, an dessen 4 Benzoresten jeweils ein weiterer Benzolkern anelliert ist, wobei eine 2,4- oder 1,2-Verknüpfung möglich ist, was den beiden Teilstrukturen

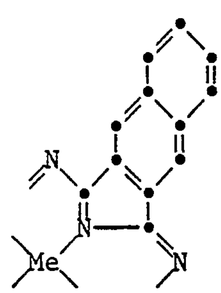 und 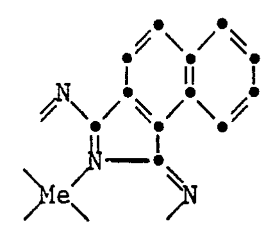 entspricht.

Z bedeutet ein beliebiges Gegenion zu einer ionischen Gruppe A. Enthält eine Gruppe A mehrere ionische Reste, kann Z die gleiche Wertigkeit wie die Gruppe A haben oder es können entsprechend viele einwertige Gegenionen Z vorhanden sein. Ist A nichtionisch, steht Z für null. Beispiele für kationische Gegenionen sind: Wasserstoffion, Alkalimetall-, Erdalkalimetall- und unsubstituierte und substituierte Ammoniumionen.

Substituierte Ammoniumionen leiten sich beispielsweise von primären, sekundären oder tertiären aliphatischen oder cyclischen Aminen ab. Beispiele hierfür sind Ammoniumionen der Formel

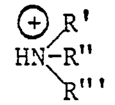 ,

worin R', R'' und R''' unabhängig voneinander Wasserstoff oder gegebenenfalls mit Halogen, Hydroxy, Phenyl oder Cyano substituiertes Alkyl (vorzugsweise mit 1-4 C-Atomen) bedeutet, wobei mindestens ein R-Substituent von Wasserstoff verschieden ist. Zwei R-Reste zusammen können auch die Ergänzung zu einem gesättigten 5- oder 6-gliedrigen Stickstoffheterocyclus bilden, der gegebenenfalls noch zusätzlich ein Sauerstoff- oder Stickstoffatom als Ringglied enthält. Beispiele für solche Heterocyclen sind: Piperidin, Piperazin, Morpholin, Pyrrolidin, Imidazolidin usw.

Bevorzugte kationische Gegenionen sind das Wasserstoffion, Alkalimetall- (insbesondere $Na^+$ und $K^+$) und Ammoniumionen.

Als Gegenionen für kationische Gruppen A kommen beliebige Anionen in Betracht, die in der Regel durch das Herstellungsverfahren (z. B. Quaternierung) eingeführt werden. Beispiele für derartige Anionen sind: Halogenionen (inklusive $J^\ominus$), Alkylsulfat- oder Arylsulfonationen wie das Benzolsulfonat-, Naphthalinsulfonat-, p-Tolylsulfonat- und das p-Chlorphenylsulfonation; ferner Sulfat-, Methylsulfat-, Sulfit-, Aminosulfonat, Bicarbonat-, Carbonat-, Perchlorat-, Phosphat-, Nitrat-, Acetat-, Propionat-, Oxalat-, Maleinat-, Citrat-, Lactat-, Succinat-, Chloracetat-, Tartrat-, Malat-, Methansulfonat- oder Benzoationen oder ein anderes Anion einer organischen Carbonsäure. Die Anionen lassen sich durch übliche Methoden leicht gegeneinander austauschen.

Als wasserlöslichmachende Gruppen A kommen, wie vorstehend erwähnt, beispielsweise anionische Gruppen in Frage. Es kommen dabei alle anionischen Gruppen oder solche Gruppen enthaltende Reste in Betracht, die den Phthalocyaninverbindungen eine hinreichende Wasserlöslichkeit vermitteln, insbesondere solche, die aus der Phthalocyaninchemie bereits bekannt sind.

Beispiele für solche anionische wasserlöslichmachende Gruppen sind Sulfo-, Carboxyl, Phosphat-, Sulfat-, Sulfinyl-, Disulfimid- und Cyanimidgruppen oder eine oder mehrere der vorgenannten Gruppen enthaltende Reste.

Beispielsweise kann A eine oder mehrere der folgenden Gruppen bedeuten:

$-SO_3^\ominus$, $-COO^\ominus$, $-SO_2^\ominus$, $-SO_2-Alk-OSO_3^\ominus$, $-SO_2-Alk-SO_3^\ominus$,

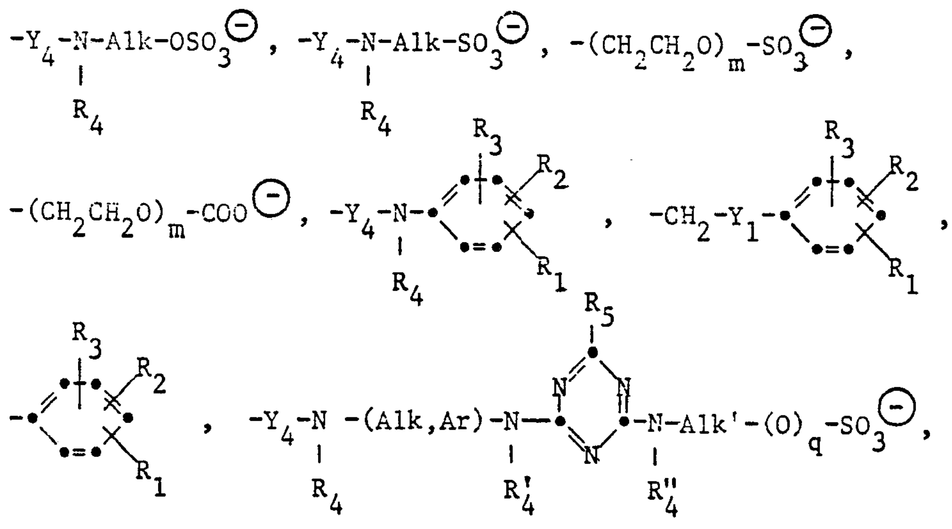

$-SO_2\overset{\ominus}{N}-CN$ oder $-SO_2\overset{\ominus}{N}-SO_2-R_6$, worin

$R^1$, $R^2$ und $R^3$ unabhängig voneinander Wasserstoff, Alkyl, Hydroxy, $SO_3^\ominus$ oder $COO^\ominus$, wobei mindestens einer dieser Substituenten für $SO_3^\ominus$ oder $COO^\ominus$ steht, $R_4$, $R'_4$ und $R''_4$ unabhängig voneinander gegebenenfalls substituiertes Alkyl oder Wasserstoff, $R_5$ Wasserstoff oder Halogen, $R_6$ gegebenenfalls substituiertes Alkyl, gegebenenfalls substituiertes Pheny] oder Naphthyl, Ar einen gegebenenfalls substituierten Phenyl- oder Naphthylrest, Alk und Alk' jeweils eine gegebenenfalls substituierte Alkylengruppe, $Y_1$ $-NR_4$ oder S, $Y_4$ $-SO_2-$

oder -CO-, vorzugsweise $-SO_2-$, m eine Zahl von 1 bis 30 und q 0 oder 1 bedeuten. $Y_4$ bedeutet dabei vorzugsweise $-SO_2-$; von den Resten $R_1$, $R_2$ und $R_3$ bedeutet $R_1$ vorzugsweise $SO_3^{\ominus}$ $R_2$ vorzugsweise Wasserstoff, $SO_3^{\ominus}$, Alkyl oder Hydroxy, insbesondere Wasserstoff, $R_3$ vorzugsweise Wasserstoff.

Sofern Phenyl- oder Naphthylringe substituiert sind, enthalten sie beispielsweise 1 bis 3 Substituenten, vorzugsweise einen Substituenten. Beispiele für solche Substituenten sind:

Alkyl oder Alkoxy, Nitro, Halogenalkyl, Halogen, Alkoxycarbonyl, Cyano, Alkylsulfonyl, Acylamino, Carboxy und deren Derivate, Sulfo und deren Derivate, Acyloxy, Trifluormethyl, Dialkylamino.

Halogen ist insbesondere Fluor, Chlor oder Brom, vorzugsweise Chlor. Als Derivate der Carboxy- und Sulfogruppen sind z. B. deren Salze, Ester und Amide zu nennen.

Sofern Alkylen- oder Alkylgruppen substituiert sind (z. B. Reste $R_4$, $R_6$, Alk), kommen als Substituenten z. B. folgende in Betracht: Hydroxy, Alkoxy, Halogen, Cyano, Aryl (insbesondere Phenyl oder Naphthyl, gegebenenfalls substituiert wie die aromatischen Gruppen $R_6$), Carbalkoxy, Aminocarbonyl oder Dialkylamino.

Alkyl-, Alkylen- und Alkoxygruppen haben als solche oder in Alkyl- oder Alkoxygruppen enthaltenden zusammengesetzten Gruppen z. B. 1 bis 8, insbesondere 1 bis 6, vorzugsweise 1 bis 4 C-Atome. Alkylreste in Carbonsäureester- oder amidgruppen oder in Sulfonamidgruppen haben vorzugsweise 1 bis 8 C-Atome. Diese bevorzugten Kettenlängen gelten für alle Alkyl-, Alkylen- und Alkoxygruppen in der vorliegenden Beschreibung, sofern nichts anderes angegeben ist.

$Y_1$ bedeutet in obigen Formeln vorzugsweise $N-R_4$. $R_4$ bedeutet vorzugsweise Wasserstoff oder unsubstituiertes Alkyl, insbesondere Wasserstoff. Alkylengruppen (Alk, Alk') sind vorzugsweise unsubstituiert.

Bevorzugte anionische Gruppen A sind: $-SO_3^{\ominus}$, $-COO^{\ominus}$, $-SO_2^{\ominus}$, $-SO_2^{\ominus}-N-CN$ oder $-SO_2^{\ominus}N-SO_2R'_6$, worin $R'_6$ für $C_1$-$C_4$-Alkyl, Phenyl, ($C_1$-$C_4$-Alkyl) phenyl, Chlorphenyl oder Methoxyphenyl steht, insbesondere die Gruppe $SO_3^{\ominus}$. Als Kation Z kommen dann vorzugsweise ein Wasserstoff-, Alkalimetall- oder unsubstituiertes oder substituiertes Ammoniumion, insbesondere ein Wasserstoff-, Natrium- oder Ammoniumion in Frage.

Als wasserlöslichmachende Gruppen A kommen auch kationische Gruppen in Betracht. Es kommen alle kationischen Gruppen oder solche Gruppen enthaltende Reste in Frage, die den Phthalocyaninverbindungen eine hinreichende Wasserlöslichkeit vermitteln, insbesondere solche, die aus der Phthalocyaninchemie bereits bekannt sind.

Beispiele für solche kationische wasserlöslichmachende Gruppen sind quaternäre Ammoniumverbindungen oder tertiäre Sulfoniumgruppen oder eine oder mehrere der vorgenannten Gruppen enthaltende Reste. Die quaternären Ammoniumverbindungen können sich von aliphatischen Aminen oder von stickstoffhaltigen aromatischen oder nichtaromatischen Heterocyclen ableiten.

So kann A beispielsweise für eine oder mehrere Gruppen der folgenden Formeln stehen:

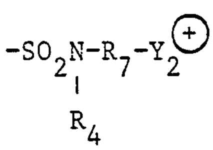, 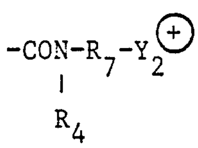, $-(CH_2)_q-Y_3^{\oplus}$ oder $-CH_2NHCOCH_2-Y_3^{\oplus}$

worin q für 0 oder 1 steht, $R_7$ unverzweigtes oder verzweigtes Alkylen mit 1 bis 8 C-Atomen oder 1,3- oder 1,4-Phenylen, $R_4$ Wasserstoff oder gegebenenfalls substituiertes Alkyl, $Y_2^{\oplus}$ eine Gruppe der Formel

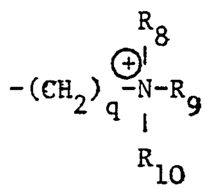, 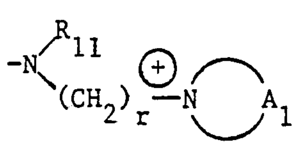, 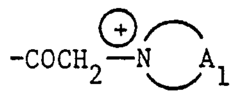;

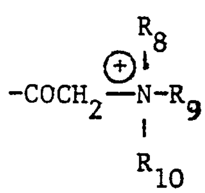

und für den Fall, dass $R_7$ = Alkylen, auch eine Gruppe der Formel

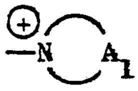 , 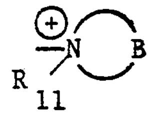 , 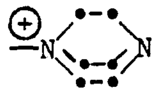 , 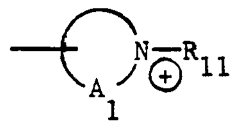 ,

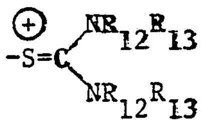 oder 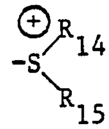 ,

$Y_3^{\oplus}$ eine Gruppe der Formel 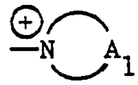 , 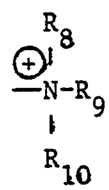 , 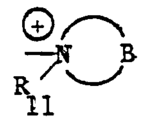 ,

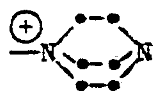 , 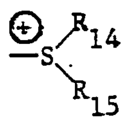 , 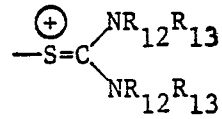 oder 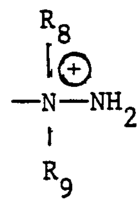

bedeuten, wobei in obigen Formeln q für 0 oder 1, $R_8$ und $R_9$- unabhängig voneinander für gegebenenfalls substituiertes Alkyl mit 1 bis 6 C-Atomen, $R_{10}$ für gegebenenfalls substituiertes Alkyl mit 1 bis 6 C-Atamen, Cycloalkyl mit 3 bis 7 C-Atomen oder die Gruppe $NR_{12}R_{13}$, $R_{11}$ für Alkyl, $R_{12}$ und $R_{13}$ unabhängig voneinander für Wasserstoff oder gegebenenfalls substituiertes Alkyl, $R_{14}$ und $R_{15}$ unabhängig voneinander für einen gegebenenfalls substituierten Alkyl- oder Aralkylrest, r für eine ganze Zahl von 1 bis 6, $A_1$ für die Ergänzung zu einem aromatischen 5 bis 7-gliedrigen Stickstoffheterocyclus, der gegebenenfalls noch ein oder zwei weitere Stickstoffatome als Ringglieder enthalten kann und der gegebenenfalls verschiedene Substituenten tragen kann, und B für die Ergänzung zu einem gesättigten 5- bis 7-gliedrigen Stickstoffheterocyclus, der gegebenenfalls noch 1 bis 2 Stickstoff-, Sauerstoff- und/oder Schwefelatome als Ringglieder enthalten kann und der gegebenenfalls verschiedene weitere Substituenten tragen kann, stehen.

Gegebenenfalls substituierte Alkylgruppen können als Substituenten beispielsweise Halogen, Hydroxyl, Cyano, Phenyl, Carboxy, Carbalkoxy oder Alkoxy enthalten. Geeignete substituierte Alkylgruppen sind Benzyl, Phenäthyl, Hydroxyalkyl und Cyanoalkyl. Cycloalkylgruppen haben vorzugsweise 5 oder 6 C-Atome, bevorzugt ist Cyclohexyl.

Als Aralkylreste kommen insbesondere Alkylreste in Betracht, die mit Phenyl, Naphthyl oder Pyridyl substituiert sind. Bevorzugt ist der Benzylrest.

Als Gruppen 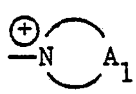 kommen vor allem in Betracht:

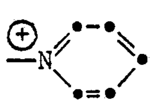 , 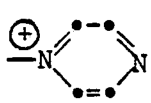 , 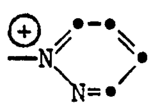 , 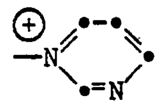 ,

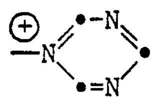 , 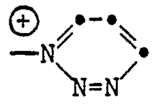 usw.

Bevorzugt ist die Gruppe 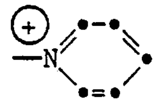 .

Als heterocyclische Ringe in der Gruppe 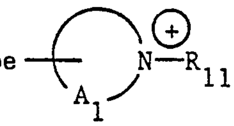 kommen ebenfalls die eben angeführten in Betracht, wobei lediglich die Bindung an den Restsubstituenten über ein Kohlenstoffatom erfolgt.

In allen Substituenten können Phenyl-, Naphthyl- und aromatische Heteroringe durch ein oder zwei weitere Reste substituiert sein, beispielsweise durch Alkyl, Alkoxy, Halogen, Carboxy, Carbalkoxy, Hydroxy, Amino, Cyano, Sulfo, Sulfonamido usw.. Bevorzugt ist ein Substituent aus der Gruppe Alkyl, Alkoxy, Halogen, Carboxy, Carbalkoxy oder Hydroxy.

Als Gruppe 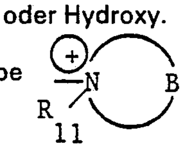 kommen insbesondere in Frage:

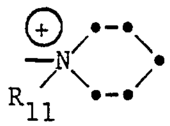 , 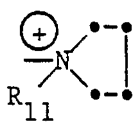 , 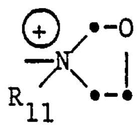 , 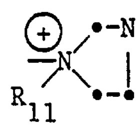 , 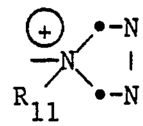 ,

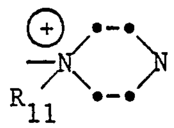 , 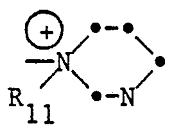 , 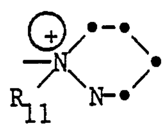 , 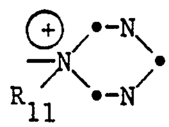 , 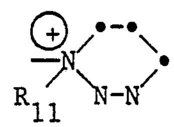 ,

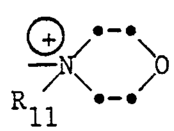 , 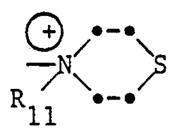 , usw.

Alle vorstehend genannten gesättigten Stickstoffheterocyclen können noch durch Alkylgruppen substituiert sein, entweder an einem Kohlenstoffatom oder an einem weiteren im Ring befindlichen Stickstoffatom. Bevorzugt ist dabei als Alkylgruppe die Methylgruppe.

Bevorzugt sind dabei Gruppen der Formel 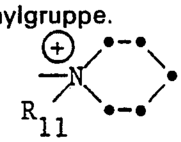 ,

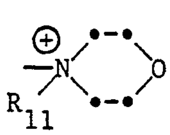 , 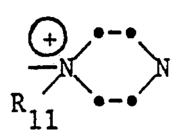 und 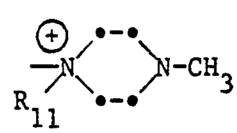 .

Die Bedeutungsmöglichkeiten für Z im Falle von A = kationische Gruppe wurden bereits weiter oben angeführt. Bevorzugte Anionen Z sind: Halogenionen (inklusive $J^{\ominus}$), Alkylsulfat- und Arylsulfonationen, z. B. ein Benzolsulfonat-, Naphthalinsulfonat-, p-Tolylsulfonat oder p-Chlorphenylsulfonation.

Halogen bedeutet in obigen Substituentendefinitionen Chlor, Brom, Fluor und Jod, besonders Chlor oder Brom, bevorzugt Chlor. $R_4$ hat dieselben bevorzugten Bedeutungen wie im Fall der anionischen Gruppen A.

A als kationische Gruppe bedeutet vorzugsweise eine solche der Formel $-SO_2NHR_7'-Y_2'^{\oplus}$, worin $R_7'$ für Alkylen mit 2 bis 6 C-Atomen und $Y_2'^{\oplus}$ für eine Gruppe der Formel

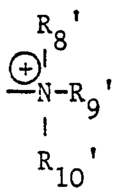 , 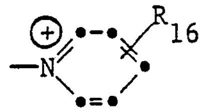 , 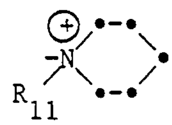 oder 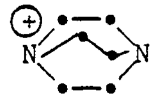

stehen, worin $R_8'$ und $R_9'$ unabhängig voneinander für unsubstituiertes oder durch Hydroxy, Cyano, Halogen oder Phenyl substituiertes Alkyl mit 1 bis 4 C-Atomen stehen, $R_{10}$ die Bedeutungsmöglichkeiten von $R_8$ hat und zusätzlich für Cyclohexyl oder die Aminogruppe stehen kann, und $R_{11}$ für $C_1$-$C_4$-Alkyl und $R_{16}$ für $C_1$-$C_4$-Alkyl, $C_1$-$C_4$-Alkoxy, Halogen, Carboxy, Carbalkoxy oder Hydroxy stehen.

Die dritte Art von Substituenten A stellen die Wasserlöslichmachenden nichtionischen Gruppen dar. Auch hier kommen alle derartigen Gruppen in Betracht, die den Phthalocyaninverbindungen eine hinreichende Wasserlöslichkeit vermitteln. Dies sind wieder vor allem solche Gruppen, die für diesen Zweck aus der Phthalocyaninchemie bekannt sind. Z is im Fall dieser Gruppen null.

Beispiele für nichtionische Gruppen A sind solche der Formeln

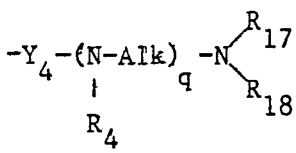 , 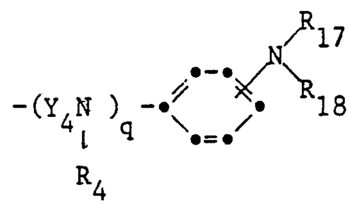 , 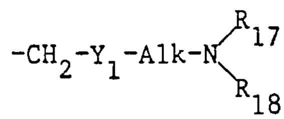 ,

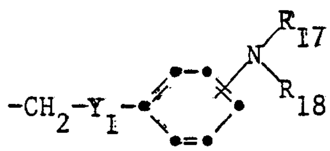 oder $-(CH_2CH_2O)_gH$

worin $Y_1$ S oder $NR_4$ $Y_4$ $-SO_2-$ oder CO-, vorzugsweise $-SO_2-$, Alk gegebenenfalls substituiertes Alkylen, $R_{17}$ und $R_{18}$ unabhängig voneinander Wasserstoff, Alkyl, Hydroxyalkyl, Cyanoalkyl, Sulfoalkyl, Carboxyalkyl oder Halogenalkyl, unsubstituiertes oder mit Halogen, Alkyl oder Alkoxy, Sulfo oder Carboxy substituiertes Phenyl oder $R_{17}$ und $R_{18}$ zusammen mit dem Stickstoffatom, an das sie gebunden sind, einen gesättigten 5- oder 6-gliedrigen heterocyclischen Ring, der zusätzlich noch ein Stickstoff- oder Sauerstoffatom als Ringglied enthalten kann, $R_4$ Wasserstoff oder gegebenenfalls substituiertes Alkyl, q 0 oder 1 und g eine Zahl von 4 bis 50 bedeuten.

Sofern Alkylen (Rest Alk) oder Alkyl (Rest $R_4$) substituiert sind, kommen als Substituenten beispielsweise Hydroxy, Alkoxy, Halogen, Cyano, Aryl (insbesondere Phenyl oder Naphthyl, gegebenenfalls substituiert wie die aromatischen Gruppen $R_6$), Carbalkoxy, Aminocarbonyl oder Dialkylamino in Frage. Vorzugsweise ist jedoch der Rest Alk bzw $R_4$ unsubstituiert. $Y_4$ bedeutet vorzugsweise $-SO_2-$, $R_4$ vorzugsweise Wasserstoff. Als heterocyclische Ringe, die $R_{17}$ und $R_{18}$ gemeinsam mit dem Stickstoffatom bilden können, sind der Morpholin-, Piperidin, Pyrazolin-, Piperazin- und Oxazolidinrest bevorzugt. $R_{17}$ und $R_{18}$ bilden vorzugsweise nur dann einen heterocyclischen Ring, wenn die Gruppe $-NR_{17}R_{18}$ an einen Alkylenrest gebunden ist. Der Index g steht vorzugsweise für eine Zahl von 8 bis 40, insbesondere 10 bis 30.

Bevorzugte nichtionische Gruppen A sind solche der Formel

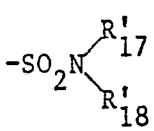 , 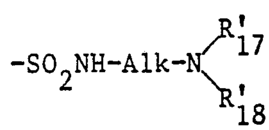 oder $-(CH_2CH_2O)_{g'}H$ ,

worin Alk $C_2$-$C_6$-Alkylen, $R'_{17}$ und $R'_{18}$ unabhängig voneinander Wasserstoff, Alkyl, Hydroxyalkyl, Cyanoalkyl oder Halogenalkyl mit jeweils 1 bis 6 C-Atomen, Phenyl oder zusammen mit dem Stickstoffatom, an das sie gebunden sind, den Piperidin-, Piperazin- oder Morpholinring und g' eine Zahl von 8 bis 40 bedeuten.

Als neutrale, nicht wasserlöslichmachende Substituenten Y sind beispielsweise zu erwähnen: Halogen (z. B. Fluor, Chlor, Brom, Jod, insbesondere Chlor oder Brom, vorzugsweise Chlor), Cyano, Nitro, gegebenenfallls substituiertes Alkyl oder gegebenenfalls substituiertes Phenyl. Alkylgruppen weisen dabei vorzugsweise 1-6,

insbesondere 1-4 C-Atome auf. Sofern solche Alkylgruppen substituiert sind, kommen als Substituenten beispielsweise Hydroxy, Alkoxy, Halogen, Cyano, gegebenenfalls substituiertes Phenyl, Carbalkoxy oder Aminocarbonyl in Betracht. Mögliche Substituenten für Phenylgruppen (auch für Phenylalkyl) sind z. B. Alkyl, Alkoxy, Nitro, Halogenalkyl, Halogen, Alkoxycarbonyl, Cyano, Alkylsulfonyl, Acylamino, Acyloxy, Trifluormethyl.

Bevorzugte Substituenten Y sind Halogen (insbesondere Chlor und Brom), Alkyl, Phenyl, Halogenphenyl, Alkylphenyl und Alkoxyphenyl, insbesondere Chlor, Brom, $C_1$-$C_4$-Alkyl oder Phenyl.

In Formel (1) bedeutet n vorzugsweise eine beliebige Zahl von 1 bis 4 und p eine beliebige Zahl von 0 bis 4. Die Zahl der wasserlöslichmachenden Substituenten, die im Molekül mindestens vorhanden sein müssen, hängt auch von der Zahl der vorhandenen Substituenten Y ab. Unabhängig davon, ob nicht wasserlöslichmachende Gruppen vorhanden sind oder nicht (p = 0), müssen in jedem Fall so viele wasserlöslichmachende Gruppen im Molekül vorhanden sein, dass eine ausreichende Wasserlöslichkeit gewährleistet ist. Es kann bereits eine Mindestlöslichkeit von 0,001 g/l ausreichend sein, im allgemeinen ist eine solche von 0,1 bis 20 g/l zweckmässig.

Die Indices n und p (sofern p nicht ohnehin null ist), können beliebige Zahlen im angegebenen Bereich darstellen. Wie in der Phthalocyaninchemie üblich, bestehen die einzelnen Produkte häufig aus Mischungen, da bei der Herstellung (z. B. Sulfonierung, Sulfochlorierung, Halogenierung usw.) oft keine einheitlichen Produkte entstehen. Die Indices zeigen daher den "Substitutionsgrad" an, der selbstverständlich nicht ganzzahlig sein muss.

Wie bereits weiter oben erwähnt, enthalten erfindungsgemässe Phthalocyanine z. B. nur eine Art von wasserlöslichmachenden Gruppen (anionische, kationische oder nichtionische). Sie können aber im Molekül auch 2 oder 3 Arten dieser Gruppen enthalten, also anionische und kationische, kationische und nichtionische, anionische und nichtionische sowie anionische, nichtionische und kationische. Nachstehend sind beispielsweise zwei Formeln von solchen gemischtsubstituierten Phthalocyaninen angegeben:

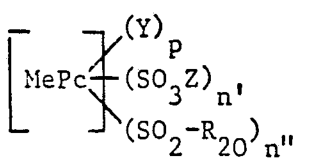

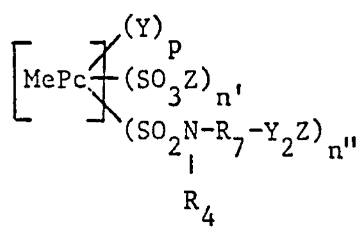
,

wobei n' + n'' = n und $R_{20}$ = Z oder -NZCN oder NZ-$SO_2$-$R_6$ und alle übrigen allgemeinen Symbole wie vorstehend definiert sind.

Besonders hervorzuheben im Rahmen der Verbindungen der Formel (1) sind jene, in denen A eine Gruppe der Formel

$-SO_3^{\ominus}$, $-SO_2^{\ominus}$, $-SO_2\overset{\ominus}{N}-CN$, $-SO_2\overset{\ominus}{N}-SO_2-R_6'$, $-SO_2NH-R_7''-SO_3^{\ominus}$,

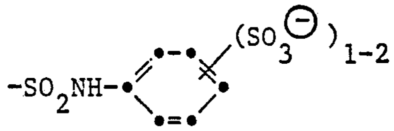
,
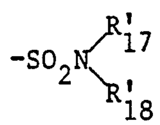
,
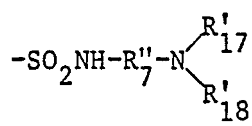
,

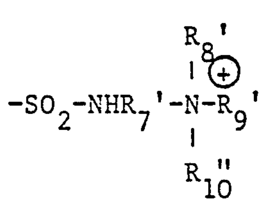
oder
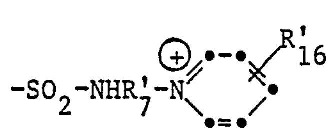

Z als Gegenion für anionische Gruppen ein Wasserstoff-, Alkalimetall-, unsubstituiertes oder substituiertes Ammoniumion, für kationische Gruppen ein Halogenid-, Alkylsulfat-, gegebenenfalls substituiertes Phenylsulfonat-, Sulfat- oder Phosphation oder das Ion einer organischen Carbonsäure und für nichtionische Gruppen 0, n eine beliebige Zahl von 1 bis 4, p eine beliebige Zahl von 0 bis 4 und Y Halogen, Alkyl oder Phenyl bedeuten, wobei in obigen Formeln $R'_6$ $C_1$-$C_4$-Alkyl- Phenyl, ($C_1$-$C_4$-Alkyl) phenyl, Chlorphenyl oder

Methoxyphenyl, $R'_7$ $C_2$-$C_6$-Alkylen, $R''_7$ $C_1$-$C_6$-Alkylen, $R'_8$, $R'_9$ und $R''_{10}$ unabhängig voneinander unsubstituiertes oder durch Hydroxy, Halogen oder Phenyl substituiertes $C_1$-$C_4$-Alkyl, $R'_{16}$ $C_1$-$C_4$-Alkyl, Halogen oder Hydroxy und $R'_{17}$ und $R'_{18}$ unabhängig voneinander Wasserstoff, Alkyl, Hydroxyalkyl, Halogenalkyl mit jeweils 1-6 C-Atomen, Phenyl oder zusammen mit dem Stickstoffatom, an das sie gebunden sind, den Piperidin-, Piperazin- oder Morpholinring bedeuten, wobei im Molekül verschiedene Arten von Substituenten A und/oder Y vorhanden sein können, insbesondere solche Verbindungen, worin A eine Gruppe der Formel $-SO_3^{\ominus}$, $-SO_2N^{\ominus}-CN$ oder $-SO_2^{\ominus}N-SO_2-R'_6$ ist, worin $R'_6$ wie vorstehend definiert ist und Z ein Wasserstoff-, Alkalimetall- oder Ammoniumion bedeutet.

Besonders bevorzugt sind dabei jene Phthalocyaninverbindungen, worin A $SO_3^{\ominus}$, Z ein Wasserstoff-, Alkalimetall- oder Ammoniumion, n eine beliebige Zahl von 1 bis 4, vorzugsweise 2 bis 4, und p eine beliebige Zahl von 0 bis 4, vorzugsweise von 0 bis 2, bedeuten.

Die erfindungsgemäßen Verbindungen der Formel (1) können nach an sich bekannten, in der Phthalocyaninchemie üblichen Verfahren hergestellt werden. Dabei kann im wesentlichen nach drei verschiedenen Methoden vorgegangen werden:

a) In ein die wasserlöslichmachenden Substituenten AZ enthaltendes metallfreies (d.h. kein Zentralatom enthaltendes) Phthalocyanin bzw. Naphthalocyanin wird das Zentralatom durch Umsetzung mit einer geeigneten Verbindung (z. B. einem Salz) des letzteren eingeführt.

b) Die wasserlöslichmachenden Substituenten werden durch geeignete Reaktionen in die entsprechenden Metallphthalocyanine bzw. -naphthalocyanine (Phthalocyanine mit Zentralatom; Phthalocyaninpigmente) eingeführt.

c) Die wasserlöslichmachenden Substituenten sind bereits in den für den Aufbau des Phthalo (bzw. Naphthalo) cyaninringsystems benötigten Ausgangsprodukten (z. B. Phthalsäureanhydrid, Phthalodinitril und die entsprechenden Naphthalindicarbonsäurederivate) enthalten. Der Aufbau des Ringsystems und der Einbau des Zentralatoms erfolgen dann entweder gleichzeitig oder nacheinander durch übliche Verfahren.

Die nicht wasserlöslichmachenden Substituenten können - je nach ihrer Natur - ebenfalls bereits in den Ausgangsprodukten enthalten sein oder sie können nachträglich, z. B. durch Halogenierung, in das aufgebaute Ringsystem eingeführt werden, und zwar vor oder nach dem Einbau des Zentralatoms. Bei manchen Herstellungsverfahren für das Phthalocyaninringsystem werden solche Substituenten (z. B. Chlor) auch direkt eingeführt, z. B. bei der Verwendung von Chloriden als Katalysatoren und Salze des einzubauenden Zentralatoms.

Wird obiges Verfahren a) angewendet, kann man beispielsweise die entsprechend substituierten Phthalocyanine bzw. Naphthalocyanine ohne Zentralatom mit einer Verbindung des entsprechenden Zentralatoms, z. B. mit einem Salz davon oder auch mit einem Alkoholat, wenn das Zentralatom ein Metall ist, das Alkoholate bildet, umsetzen. Als Lösungsmittel kommen dabei z. B. Gemische aus Wasser und organischen Lösungsmitteln, etwa tertiäre Amine, oder wasserfreie Lösungsmittel wie z. B. Pyridin und Chlorbenzole in Betracht. Erhaltene Metallkomplexe können selbstverständlich auch in andere Metallkomplexe übergeführt werden. Die Verfahrensvariante a) ist z. B. auch in der US-A-4 318 883 beschrieben.

Die Methoden zur Einführung von Substituenten in das Ringsystem (obige Möglichkeit b) sind sehr vielfältig und variieren je nach Art der Substitution. Im folgenden seien nur einige Beispiele für solche Methoden angegeben:

Die Einführung von Sulfonsäuregruppen ($A = SO_3^{\ominus}$) kann beispielsweise durch Sulfonierung, etwa mit Hilfe von Oleum erfolgen. Alternativ können entsprechende unsulfonierte Phthalocyanine auch mit Chlorsulfonsäure zu den entsprechenden Phthalocyaninsulfochloriden umgesetzt und anschliessend zu den Sulfonsäuren hydrolysiert werden. In beiden Fällen können die freien Sulfonsäuregruppen nachträglich in ihre Salze übergeführt werden. Derartige Sulfonierungsmethoden sind z. B. in der US-A-4 318 883 und der EP-A-47 716 beschrieben.

Carboxylgruppen lassen sich in die unsubstituierten Phthalocyanine durch Umsetzung mit Phosgen und Aluminiumchlorid und Hydrolyse des gebildeten Säurechlorids oder durch Umsetzung mit Trichloressigsäure einführen. Die Säurechloride können in bekannter Weise auch in andere wasserlösliche Carbonsäurederivate übergeführt werden. Mit Carboxylgruppen substituierte Phthalocyanine lassen sich auch durch Synthese aus Trimellithsäure herstellen.

Aus den weiter oben beschriebenen $SO_2$-Cl bzw. COCl- substituierten Phthalocyaninen (erhalten durch Umsetzung mit Chlorsulfonsäure bzw. mit Phosgen und $AlCl_3$) erhält man durch Umsetzung mit entsprechend substituierten aliphatischen oder aromatischen Aminen die mit Sulfonamid- bzw. Carbonsäureamidgruppen vom Typ der Formeln

$-Y_4-N(R_4)-Alk-OSO_3^{\ominus}$, $-Y_4-N(R_4)-Alk-SO_3^{\ominus}$, 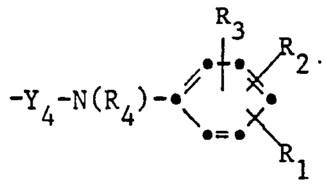 ,

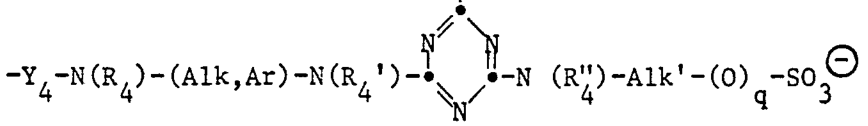 ,

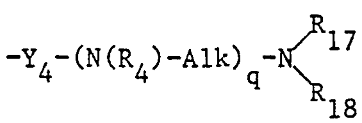 und 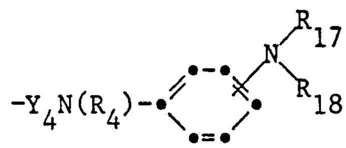

substituierten Verbindungen der Formel (1).

Verbindungen der Formel (1), die durch Gruppen der Formeln

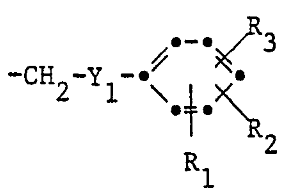 , 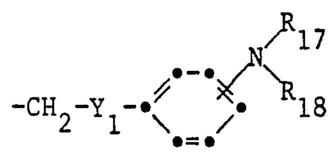

und

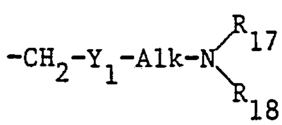 substituiert sind, können durch Chlormethylierung von unsubstituierten metallfreien oder metallisierten Phthalocyaninen, z. B. durch Umsetzung mit Paraformaldehyd oder Bis-chlormethyläther und wasserfreiem Aluminiumchlorid in Gegenwart von Triäthylamin, und anschliessender Umsetzung der Chlormethylverbindungen mit entsprechend substituierten Anilinen oder Thiophenolen bzw. Aminen oder Mercaptanen erhalten werden.

Verbindungen der Formel (1), die mit wasserlöslichmachenden Gruppen der Formeln

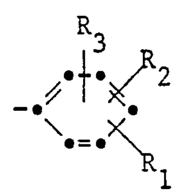 oder 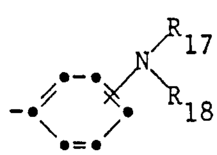

substituiert sind, können beispielsweise erhalten werden, wenn man von entsprechend substituiertem Phthalsäureanhydrid oder Phthalodinitril (oder den entsprechenden Naphthalindicarbonsäurederivaten)

ausgeht und diese in bekannter Weise zum Phthalocyaninringsystem umsetzt. Bei Verwendung von substituierten Phthalodinitril wird dieses, gegebenenfalls zusammen mit einem Metallsalz, geschmolzen oder in Lösung oder Suspension zum Phthalocyaninringsystem cyclisiert. Bei Verwendung des entsprechenden Phthalsäureanhydrids wird zusätzlich Harnstoff und gegebenenfalls ein Katalysator, wie z. B. Borsäure oder Ammoniummolybdat vor der Reaktion zugegeben. Auch andere substituierte Phthalocyanine, z. B. auch die sulfonierten Phthalocyanine, können auf diese Weise erhalten werden.

Die vorstehend genannten Verfahren sind auch in der US-A-4 318 883 beschrieben.

Verbindungen der Formel (1), worin A eine Gruppe der Formel $-SO_2^{\ominus}N-CN$, $SO_2^{\ominus}N-SO_2-R_6$ oder $-SO_2^{\ominus}$ bedeutet, stellt man beispielsweise her durch Umsetzung des entsprechenden Phthalocyanins (Naphthalocyanins) mit Chlorsulfonsäure und weitere Umsetzung des erhaltenen Sulfochlorids mit Cyanamid, bzw. mit Ammoniak und einem Halogenid der Formel $Hal-SO_2-R_6$ (Hal = Halogen, insbesondere Chlor) bzw. mit Hydrazin oder einem Hydrazinderivat. Die entsprechenden Verfahren sind z. B. in der EP-A-81 462 beschrieben.

Zur Herstellung von Verbindungen der Formel (1), die $-(CH_2CH_2O)_m-SO_3^{\ominus}$ oder $-(CH_2CH_2O)_gH$-Gruppen enthalten, geht man beispielsweise von mit Hydroxygruppen substituierten, bereits metallisierten oder noch metallfreien Phthalocyaninen oder Naphthalocyaninen aus und setzt sie mit Aethylenoxid um. Das Umsetzungsprodukt kann dann z. B. mit Schwefelsäure verestert werden. Aus den genannten hydroxysubstituierten Phthalocyaninen kann man auch Verbindungen der Formel (1) herstellen, die Sulfat-, Carboxylat-, Methoxycarboxylat-, Polyäthoxycarboxylat-, Phosphat- und Polyäthoxyphosphatgruppen enthalten. Derartige Verfahren sind in der EP-A-3 149 erwähnt.

Die Einführung von kationischen Substituenten in das Phthalocyanin- bzw. Naphthalocyaninringsystem, das bereits ein Zentralatom enthalten kann oder in das letzteres erst nach der Einführung der Substituenten eingebaut wird (obige Methode a), erfolgt ebenfalls nach in der Phthalocyaninchemie üblichen Methoden.

Zur Herstellung von Verbindungen der Formel (1), worin A eine Gruppe $-SO_2N(R_4)-R_7-Y_2^{\oplus}$ oder $-CON(R_4)-R_7-Y_2^{\oplus}$ bedeutet, setzt man beispielsweise das jeweilige Phthalocyanin mit Chlorsulfonsäure zu den entsprechenden Sulfochloridverbindungen bzw. mit Phosgen und $AlCl_3$ zu den entsprechenden Carbonsäurechloridverbindungen um. Diese Sulfochlorid- bzw. Carbonsäurechlorid-Phthalocyanine lässt man dann in bekannter Weise mit einem Diamin bzw. Mercapto-Amin der Formel

(2) 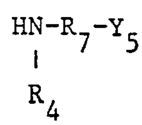 bzw. 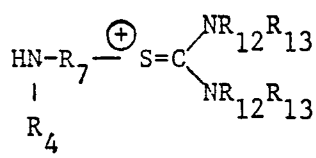 (3) oder

(4) 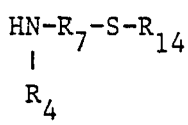

reagieren, wobei $R_7$, $R_{12}$, $R_{13}$ wie oben definiert sind und $Y_5$ eine noch nicht quaternierte tertiäre Aminogruppe bedeutet. In den so erhaltenen Sulfonamido-Phthalocyaninen wird die tertiäre Aminogruppe $Y_5$ nach bekannten Methoden zu einer Gruppe $Y_2^{\oplus}$ quaterniert oder die Mercaptogruppe in Formel (4) zu der Gruppe

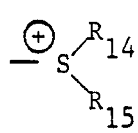

terniert.

Die Phthalocyanin-Sulfochloride bzw. -Carbonsäurechloride lassen sich jedoch auch in bekannter Weise mit Aminen umsetzen, die bereits quaternäre Ammoniumgruppen bzw. ternäre Sulfoniumgruppen enthalten (also Umsetzung mit Aminen der Formel (2), worin $Y_5$ durch $Y_2^{\oplus}$ ersetzt ist bzw. das Schwefelatom im Amin der Formel (4) bereits terniert ist).

Eine weitere Methode besteht darin, die Phthalocyanin-Sulfochloride bzw. -Carbonsäurehalogenide mit Halogen-Aminen der Formel

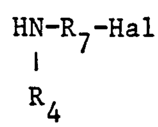 (5)

(Hal = Halogen) und die resultierenden Phthalocyanin-Sulfonamid- bzw. -Carbonsäureamid-Verbindungen z. B. mit einem tertiären Amin umzusetzen. Werden Verbindungen der Formel (1) gewünscht, die neben den kationischen Gruppen auch noch freie Sulfo- bzw. Carboxylgruppen enthalten, so setzt man die Phthalocyanin-Sulfochloride bzw. -Carbonsäurechloride mit unterstöchiometrischen Mengen an Aminen der Formeln (2) bis (5) um und hydrolysiert, gegebenenfalls nach Quaternisierung, die restlichen freien $SO_2Cl$- bzw. COCl-Gruppen zu den Sulfo- bzw. Carboxylgruppen oder deren Salzen.

Verbindungen der Formel (1) mit Substituenten der Formel $-(CH_2)_q-Y_3^{\oplus}$, worin q = 1, können aus den entsprechenden, durch Chlormethylierung erhaltenen Chlormethyl-Phthalocyaninen durch Umsetzung beispielsweise mit einem tertiären Amin erhalten werden. Verbindungen der genannten Art, in denen q = 0 können in ähnlicher Weise aus chlorierten Phthalocyaninen hergestellt werden.

Alternativ können die vorgenannten Verbindungen in der Weise erhalten werden, indem man von einem entsprechend substituierten Phthalsäureanhydrid oder Phthalodinitril (bzw. den entsprechenden Derivaten von Naphthalindicarbonsäuren) ausgeht und diese in bekannter Weise zum Phthalocyanin- bzw. Naphthalocyaninringsystem kondensiert. Bei Verwendung von substituiertem Phthalodinitril (und des entsprechenden Naphthalinderivates) wird dieses, gegebenenfalls zusammen mit einem Metallsalz, geschmolzen oder in Lösung oder Suspension zum Phthalocyanin- bzw. Naphthalocyaninringsystem cyclisiert. Bei Verwendung des entsprechenden Phthalsäureanhydrids (und des entsprechenden Naphthalinderivates) wird zusätzlich Harnstoff und gegebenenfalls ein Katalysator, wie z. B. Borsäure oder Ammoniummolybdat vor der Reaktion zugegeben.

Bei den letztgenannten Methoden können die quaternären Ammonium bzw. ternären Sulfoniumgruppen bereits in den Phthalsäure-Ausgangsderivaten vorhanden sein oder die entsprechenden tertiären Amine oder die entsprechenden Mercaptane können nachträglich im aufgebauten Phthalocyaninringsystem quaterniert oder terniert werden.

Sofern die vorgängig beschriebenen Substitutionsreaktionen nicht direkt mit den Metallkomplexen bzw. die Aufbaureaktionen des Phthalocyaninringsystems nicht in Gegenwart einer Verbindung des Zentralatomes Me ausgeführt werden, kann ein entsprechend substituiertes metallfreies Phthalocyanin nachträglich mit einem Salz des Zentralatoms Me oder dessen Alkoholat in einem Lösungsmittel umgesetzt werden. Als Lösungsmittel kommen z. B. Gemische aus Wasser und organischen Lösungsmitteln, insbesondere auch tertiäre Amine oder auch wasserfreie organische Lösungsmittel, z. B. Pyridin oder Chlorbenzole, in Betracht.

Die vorstehend skizzierten Reaktionen zur Herstellung von kationische Gruppen enthaltenden Phthalocyaninen sind ausführlicher in den EP-A-3 149 und 35 470 beschrieben. Die erfindungsgemässen Verbindungen mit kationischen Gruppen können in Analogie zu den dort beschriebenen Verfahren hergestellt werden.

Der Aufbau des Phthalocyaninringgerüstes aus Derivaten der Phthalsäure, wobei chlorierte Phthalocyanine entstehen, ist in Ullmann's Encyclopädie der technischen Chemie, 4. Aufl., Band 18, Seite 507 ff und bei F.H.Moser, A.L .Thomas, "Phthalocyanine" (1963) , Seite 104 ff beschrieben. Die dort beschriebenen Verfahren gelten auch für den Aufbau des Naphthalocyaninringgerüstes. Wie bereits früher erwähnt, können manche wasserlöslichmachende Substituenten schon in den Ausgangsverbindungen vorhanden sein oder sie können nachträglich in das wie beschrieben aufgebaute, noch unsubstituierte bzw. schon Substituenten Y enthaltende Ringsystem eingeführt werden. Es ist selbstverständlich, dass man die vorstehend genannten Methoden beliebig kombinieren kann, so dass Verbindungen der Formel (1) entstehen, die verschiedenartige Substituenten A und/oder Y im Molekül enthalten.

Die unsubstituierten Phthalocyanine (Pigmente) sind ausserdem aus der Literatur bekannt. Für ihre Herstellung siehe auch den nachstehenden Beispielteil.

Die erfindungsgemässen Verbindungen der Formel (I) zeigen ausgezeichnete photosensibilisierende Eigenschaften und sind hervorragende Singlett-Sauerstoff-Produzenten. Sie können daher erfindungsgemäss als Photosensibilisatoren, Photoaktivatoren (diese beiden Begriffe werden in der Literatur häufig synonym gebraucht) und/oder Singlett-Sauerstoff-Produzenten verwendet werden. Die Anwendungsgebiete sind sehr vielfältig. Die Verbindungen der Formel (1) können überall dort eingesetzt werden, wo photokatalysierte Reaktionen, insbesondere mit Hilfe von Singlett-Sauerstoff, ablaufen sollen. Dies können beispielsweise photokatalysierte Reaktionen in der organischen Chemie sowie auch in der Polymerchemie sein. Die erfindungsgemässen Verbindungen weisen überraschenderweise in mancher Hinsicht bessere bzw. günstigere Eigenschaften auf als bekannte Photoaktivatoren.

Bevorzugt werden die erfindungsgemässen Verbindungen jedoch als photodynamisch wirksame Mittel (d.h. als Mittel, die unter Einwirkung von Licht vor allem gegen Mikroorganismen wirken) und insbesondere als Photobleichmittel verwendet. Sie werden daher beispielsweise zum Bleichen von bzw. Fleckenentfernen aus Textilien und zum Bekämpfen von Mikroorganismen in oder auf organischen oder anorganischen Substraten bzw. zum Schützen derselben vor dem Befall durch Mikroorganismen verwendet, insbesondere als Bleichmittel bzw. antimikrobielle Wirkstoffe in Waschmitteln und Waschlaugen, aber auch als Desinfektionsmittel für Wäsche, feste Oberflächen, Schwimmbecken und Effluenten aus Kläranlagen.

Die vorliegende Erfindung betrifft auch ein Verfahren zur Durchführung einer photosensibilisierten (photoaktivierten) bzw. durch Singlett-Sauerstoff katalysierten Reaktion, das dadurch gekennzeichnet ist, dass man ein oder mehrere Phthalocyaninverbindungen der Formel (1) in Gegenwart von Sauerstoff mit dem Medium, in oder an dem die genannte Reaktion stattfinden soll, in Berührung bringt oder in dieses Medium einverleibt und mit Licht bestrahlt.

Insbesondere betrifft die vorliegende Erfindung ein Verfahren zum Bleichen von bzw. Fleckenentfernen aus Textilien und zum Bekämpfen von Mikroorganismen in oder auf organischen oder anorganischen Substraten bzw. zum Schützen derselben vor dem Befall durch Mikroorganismen, das dadurch gekennzeichnet ist, dass

man die Textilien bzw. die von Mikroorganismen zu befreienden oder zu schützenden Substrate mit in Formel (1) definierten Phthalocyaninen in Gegenwart von Wasser und unter Bestrahlung mit Licht behandelt.

Zur Entfaltung ihrer antimikrobiellen Aktivität benötigen die erfindungsgemässen Phthalocyaninverbindungen die Gegenwart von Sauerstoff und Wasser sowie eine Bestrahlung durch Licht. Man arbeitet daher im allgemeinen in wässrigen Lösungen oder auf feuchten Substraten, als Sauerstoffquelle dient der im Wasser gelöste Sauerstoff oder der Luftsauerstoff.

Die Bestrahlung kann mit einer künstlichen Lichtquelle oder durch Sonnenlicht erfolgen. Gute Wirkung wird z. B. durch Licht im Bereich zwischen etwa 300 und 2500 nm erzielt. So kann beispielsweise mit einer handelsüblichen Glühlampe bestrahlt werden. Die Beleuchtungsintensität kann in weiten Grenzen schwanken. Sie richtet sich nach der Konzentration an Wirkstoff, nach der Beschaffenheit des Substrates bzw. den zusätzlich vorhandenen Stoffen, durch die die Lichtausbeute beeinflusst wird. Als weiterer Parameter kann die Belichtungszeit variiert werden, d.h. bei geringerer Lichtintensität muss für die gleiche Wirkung länger belichtet werden als bei grösserer Intensität. Im allgemeinen sind, je nach Anwendungsgebiet, Belichtungszeiten von einigen Minuten bis zu einigen Stunden möglich.

Wird das Verfahren in einem wässrigen Bad durchgeführt (z. B. Entkeimung von Textilien), kann die Bestrahlung mit Licht entweder direkt im Behandlungsbad durch eine innerhalb oder ausserhalb desselben angebrachte künstliche Lichtquelle erfolgen oder die Substrate können nachträglich in feuchtem Zustande entweder ebenfalls mit einer künstlichen Lichtquelle bestrahlt oder dem Sonnenlicht ausgesetzt werden.

Bereits mit sehr geringen Konzentrationen an Wirksubstanz, z. B. mit 0,001 ppm können gute antimikrobielle Effekte erzielt werden. Bevorzugt ist, je nach Anwendungsgebiet und je nach eingesetztem Phthalocyaninderivat, eine Konzentration zwischen 0,005 und 100, vorzugsweise 0,01 und 50 ppm. Da die Wirksubstanzen Farbstoffe sind, ist die obere Konzentrationsgrenze dadurch gegeben, dass bei deren Ueberschreitung eine unerwünschte Anfärbung der Substrate zu beobachten wäre. Die obere Konzentrationsgrenze ist also durch die Stärke der Eigenfarbe der eingesetzten Mittel begrenzt, kann aber 1000 ppm und mehr betragen.

Die im erfindungsgemässen Verfahren eingesetzten Phthalocyaninverbindungen der Formel (1) weisen ein ausserordentlich breites Spektrum von Wirksamkeit gegen Mikroorganismen auf. So können durch das erfindungsgemässe Verfahren vor allem grampositive, aber auch gramnegative Bakterien bekämpft werden bzw. verschiedene Substrate vor dem Befall durch diese geschützt werden. Eine Wirkung wird auch gegen Pilze und Hefen beobachtet.

Im erfindungsgemässen Verfahren können zusätzlich wirkungssteigernde Substanzen zugesetzt werden, unter anderem Elektrolyte, z. B. anorganische Salze, etwa Natriumchlorid, Kaliumchlorid, Natriumsulfat, Kaliumsulfat, Natriumacetat, Ammoniumacetat, Alkalimetallphosphate, Alkalimetalltripolyphosphate, insbesondere Natriumchlorid und Natriumsulfat. Diese Salze können den erfindungsgemässen Mitteln oder direkt beim Applikationsverfahren zugesetzt werden, sodass sie in einer Konzentration von vorzugsweise 0,1 bis 10 % in der Applikationslösung vorhanden sind.

Aufgrund des erwähnten breiten Wirkungsspektrums gegen Mikroorganismen kann das erfindungsgemässe Verfahren bzw. die erfindungsgemässen Mittel in einer Reihe von Anwendungsgebieten eingesetzt werden, die nachstehend beispielsweise angeführt sind.

Als wichtige Applikation sei die Entkeimung von Textilien synthetischer oder natürlicher Herkunft erwähnt. So kann Waschgut im Haushalt oder in der Industrie mit Hilfe des erfindungsgemässen Verfahrens desinfiziert werden. Das Waschgut kann dazu mit wässrigen Lösungen der erfindungsgemässen Phthalocyaninderivate unter Bestrahlung mit Licht in der oben erwähnten Weise behandelt werden. In der Behandlungsflotte kann der Phthalocyaninfarbstoff vorteilhaft in einer Konzentration von 0,01 bis 50 mg/l enthalten sein. Die Entkeimung kann mit Vorteil auch zusammen mit dem Waschvorgang durchgeführt werden. Zu diesem Zweck wird das Waschgut mit einer Waschflotte behandelt, die übliche Waschaktivsubstanzen, ein oder mehrere erfindungsgemässe Phthalocyaninderivate und gegebenenfalls anorganische Salze und/oder weitere antimikrobiell wirksame Substanzen enthält. Der Waschvorgang kann dabei manuell, z .B. in einem Bottich oder auch in einer Waschmaschine durchgeführt werden. Die nötige Belichtung kann während des Waschvorganges durch geeignete Lichtquellen erfolgen oder das feuchte Waschgut kann auch nachträglich, z. B. während des Trocknens entweder mit einer geeigneten künstlichen Lichtquelle belichtet oder auch einfach dem Sonnenlicht ausgesetzt werden.

Die Verbindungen der Formel (1) können der Desinfektions- bzw. Bleich- oder Waschflotte direkt zugesetzt werden. Sie können aber auch in Seifen oder Waschmittel eingearbeitet werden, die bekannte Mischungen von Waschaktivsubstanzen, wie beispielsweise Seife in Form von Schnitzeln und Pulver, Synthetika, lösliche Salze von Sulfonsäurehalbestern höherer Fettalkohole, höher und/oder mehrfach alkylsubstituierten Arylsulfonsäuren, Sulfocarbonsäureester mittlerer bis höherer Alkohole, Fettsäureacylaminoalkyl- oder -aminoarylglycerinsulfonate, Phosphorsäureester von Fettalkoholen usw., Aufbaustoffe, sogenannte "Builders", z. B. Alkalipoly- und -polymetaphosphate, Alkalipyrophosphate, Phosphatersatzstoffe und Additive wie Alkalisalze der Carboxymethylcellulose und andere "Soil-redepositionsinhibitoren", ferner Alkalisilikate, Nitrilotriessigsäure, Aethylendiaminotetraessigsäure, Schaumstabilisatoren wie Alkanolamide höherer Fettsäuren sowie gegebenenfalls antistatische Mittel, rückfettende Hautschutzmittel wie Lanolin, Enzyme, Parfüme und Farbstoffe, optische Aufheller, weitere anorganische Salze und/oder weitere antimikrobielle Wirkstoffe bzw. Bleichmittel enthalten.

Das erfindungsgemässe Verfahren kann auch zur antimikrobiellen Ausrüstung von Textilien verwendet

werden, da die erfindungsgemässen Phthalocyaninderivate gut auf die Faser aufziehen und einen lang anhaltenden Effekt gewährleisten.

Ein weiteres Anwendungsgebiet des erfindungsgemässen Verfahrens und der erfindungsgemässen Mittel ist die Desinfektion von Spitalwäsche, medizinischen Gebrauchs- und Ausrüstungsgegenständen sowie von Böden, Wänden und Mobiliar (Oberflächendesinfektion) im allgemeinen sowie auch besonders in Spitälern. Die Desinfektion von Spitalwäsche kann in der für allgemeines Waschgut oben beschriebenen Weise erfolgen. Die übrigen Gegenstände sowie Boden- und Wandflächen können mit wässrigen Lösungen, die erfindungsgemässe Phthalocyaninverbindungen enthalten, behandelt werden und dabei oder nachträglich mit geeigneten Lichtquellen belichtet werden. Die Desinfektionslösungen können zusätzlich noch waschaktive Substanzen, andere antimikrobiell wirksame Verbindungen und/oder anorganische Salze enthalten.

Zur Oberflächendesinfektion kann beispielsweise auf die betreffende Oberfläche eine wässrige Lösung der erfindungsgemässen Phthalocyaninverbindungen aufgebracht werden (z. B. durch Aufsprühen), welche Lösung vorzugsweise etwa 0,001-50 ppm an Wirksubstanz enthält. Die Lösung kann auch noch andere übliche Zusätze enthalten, z. B. Netz-, Dispergier- oder Emulgiermittel, Waschaktivsubstanzen und gegebenenfalls anorganische Salze. Die Oberfläche wird nach dem Aufbringen der Lösung einfach dem Sonnenlicht ausgesetzt oder es kann bei Bedarf zusätzlich mit einer künstlichen Lichtquelle, z. B. einer Glühlampe, bestrahlt werden. Es empfiehlt sich, während der Belichtung die Oberfläche feucht zu halten.

Das erfindungsgemässe Verfahren bzw. die erfindungsgemässen Mittel können vorteilhaft auch zur Entkeimung bzw. Desinfektion von Schwimmbädern eingesetzt werden. Dem Wasser im Schwimmbad wird dazu zweckmässig eine oder mehrere der Verbindungen der Formel (1), vorzugsweise in einer Menge von 0,001 bis 50, insbesondere von 0,01 bis 10 ppm, zugesetzt. Die Belichtung erfolgt einfach durch das Sonnenlicht. Gegebenenfalls kann eine zusätzliche Belichtung durch eingebaute Lampen vorgesehen werden. Durch das beschriebene Verfahren kann man das Wasser von Schwimmbecken frei von unerwünschten Keimen halten und eine ausgezeichnete Wasserqualität aufrechterhalten.

Auch zur Entkeimung von Effluenten aus Kläranlagen kann das erfindungsgemässe Verfahren angewendet werden. Dazu setzt man dem Effluenten beispielsweise 0,001-100, insbesondere 0,01-10 ppm einer oder mehrerer der Verbindungen der Formel (1) zu. Die Bestrahlung erfolgt zweckmässig durch das Sonnenlicht, gegebenenfalls kann zusätzlich mit künstlichen Lichtquellen bestrahlt werden.

Die vorerwähnten Anwendungsmöglichkeiten stellen nur eine beispielhafte Aufzählung für die sehr breite Anwendbarkeit des erfindungsgemässen Verfahrens und damit der erfindungsgemässen Phthalocyanine der Formel (1) dar.

Besonders bevorzugt ist das erfindungsgemässe Verfahren zum Bleichen von und Fleckenentfernen aus Textilien.

Das erfindungsgemässe Bleich- und Fleckenentfernungsverfahren, in dem die erfindungsgemässen Phthalocyaninverbindungen verwendet werden, d.h. die Behandlung von Textilien mit diesen Verbindungen, wird bevorzugt in einem wässrigen Bad, insbesondere in neutralem oder alkalischem pH-Bereich ausgeführt.

Die erfindungsgemässen Phthalocyanine werden mit Vorteil in Mengen von 0,01 bis 100, insbesondere 0,01 bis 50 mg/l Behandlungsbad eingesetzt, wobei die eingesetzte Menge je nach Anzahl der wasserlöslichmachenden Gruppen und des Substituenten $R_2$ variieren kann.

Das Verfahren wird vorzugsweise als kombiniertes Wasch- und Bleichverfahren ausgeführt, in welchem Falle das wässrige Bad auch ein organisches Waschmittel, wie Seife oder synthetische Waschmittel (Waschativsubstanzen) enthält, und gegebenenfalls auch andere Waschmittelzusätze, wie Schmutzsuspendiermittel, beispielsweise Natriumcarboxymethylcellulose, Komplexbildner wie Natriumtripolyphosphat, Natriumsilikat und Natriumäthylendiamintetraacetat und optische Aufhellungsmittel enthalten kann. Als Waschaktivsubstanzen kommen z. B. jene in Betracht, die weiter oben in Zusammenhang mit dem Einsatz der Verbindungen der Formel (1) in Desinfektions- bzw. Bleich- oder Waschflotten und den zugehörigen Waschmitteln aufgezählt wurden. Das erfindungsgemässe Phthalocyanin kann daher entweder bereits in das entsprechende Waschmittel eingearbeitet sein oder kann nachträglich zur Waschflotte zugesetzt werden. Das Verfahren kann allerdings auch als reines Bleichverfahren ohne Waschmittelzusätze ausgeführt werden. In diesem Falle ist es vorteilhaft, dass das Behandlungsbad einen Elektrolyt, z. B. Natriumchlorid, Natriumsulfat oder Natriumtripolyphosphat enthält, um das Aufziehen des Phthalocyaninfarbstoffes zu gewährleisten. Die Mengen an Elektrolyt können etwa 0,5 bis 20 g/l betragen.

Wie oben erwähnt, können die Wasch- bzw. Bleichflotten gegebenenfalls auch einen oder mehrere optische Aufheller enthalten. Dies können übliche Waschmittelaufheller sein. Bevorzugt werden aber Aufheller aus den Klassen der Distyrylbiphenylsulfonsäuren und deren Salzen und/oder der 4,4'-Bis-(1,2,3-triazol-2-yl)stilbendisulfonsäuren und deren Salzen eingesetzt. Mit Hilfe dieser Aufheller werden in Kombination mit den erfindungsgemässen Photoaktivatoren ganz besonders gute Bleicheffekte erzielt, die höher sind als aus der additiven Wirkung der Einzelkomponenten zu erwarten wäre. Als derartige Aufheller kommen insbesondere solche der weiter unten angegebenen Formel (A1), z. B. der Formel (A2), ganz besonders bevorzugt der Formel (A3) im Betracht. Auch Aufheller der Formel (A4) ergeben gute Resultate, ebenso wie Mischungen aus den Aufhellern der Formeln (A3) und (A4).

Das erfindungsgemässe Bleichverfahren wird zweckmässigerweise bei Temperaturen im Bereich von etwa 20 bis 100, insbesondere von 20 bis 85°C, während eines Zeitraumes von 15 Minuten bis 5 Stunden, vorzugsweise 15 Minuten bis 60 Minuten, ausgeführt.

Für das erfindungsgemässe Bleichverfahren ist die Anwesenheit von Sauerstoff und die Bestrahlung mit

Licht erforderlich. Als Sauerstoffquelle genügt der im Wasser gelöste bzw. in der Luft vorhandene Sauerstoff.

Die Bestrahlung kann mit einer künstlichen Lichtquelle (z. B. Glühlampe, Infrarotlampe) erfolgen, wobei das Bleich- bzw. Waschbad direkt bestrahlt werden kann, sei es durch eine Lichtquelle innerhalb des Behälters, in dem sich die Flotte befindet (z. B. Lampe in der Waschmaschine), sei es durch eine Lichtquelle ausserhalb des Behälters. Ebenso kann die Bestrahlung aber auch erst nach der Entnahme der Textilien aus dem Behandlungsbad erfolgen. In diesem Fall sollen die Textilien jedoch noch feucht sein bzw. sie müssen nachtraglich wieder befeuchtet werden. Als Lichtquelle kann aber besonders vorteilhaft Sonnenlicht dienen, wobei die Textilien entweder während einer Behandlung im Einweichbad, oder nach der Behandlung im Wasch- bzw. Bleichbad in feuchtem Zustand dem Sonnenlicht ausgesetzt werden. Vorzugsweise soll die verwendete Lichtquelle Licht im Wellenlängenbereich von 300-800 nm liefern.

Obwohl die Verbindungen der Formel (1) im allgemeinen sehr gute Bleicheffekte liefern, sind für den Einsatz in üblichen Wasch-, Bleich- und Einweichmitteln, die üblicherweise anionische und/oder nichtionische waschaktive Substanzen enthalten, die mit anionischen Gruppen substituierten Verbindungen der Formel (1) (A anionische Gruppen) bevorzugt, insbesondere jene mit A = $SO_3^{\ominus}$, $COO^{\ominus}$, $SO_2^{\ominus}$, $SO_2^{\ominus}NCN$ und $SO_2^{\ominus}N\text{-}SO_2\text{-}R_6$, vor allem jene mit A = $SO_3^{\ominus}$. Selbstverständlich können in solchen Waschmitteln auch Verbindungen der Formel (1) eingesetzt werden, die nichtionische Gruppen A enthalten.

Erfindungsgemässe Verbindungen der Formel (1), die kationische Wasserlöslichmachende Gruppen enthalten, werden, sofern als Photobleichmittel für Textilien verwendet, vorzugsweise zusammen mit kationischen Textilbehandlungsmitteln eingesetzt, z. B. mit kationischen Tensiden, Weichmachern, Antistatika, optischen Aufhellern oder/und Antimikrobika. Diese kationischen Textilbehandlungsmittel können beispielsweise der Wasch-, Bleich-, Spül-, Einweich- oder Nachbehandlungsflotte getrennt vom kationischen Photobleichmittel zugegeben werden. Sie können aber auch gemeinsam mit letzterem bereits in einem Textilbehandlungsmitte, insbesondere einem Wasch-, Bleich-, Einweich- oder Spülmittell, inkorporiert sein. Die einzelnen kationischen Bestandteile, die vorzugsweise gemeinsam mit einem kationischen Photobleichmittel der Formel (1) eingesetzt werden können, sind weiter unten bei den entsprechenden Mitteln beschrieben.

Das erfindungsgemässe Verfahren zum Bleichen mit Hilfe von Verbindungen der Formel (1) kann auch in Gegenwart von Reduktionsmitteln ausgeführt werden. Diese "reduktive" Bleiche kann in einigen Fällen (je nach Substrat, Schmutzart usw.) eine Verbesserung des Bleicheffekts bringen. Vorzugsweise wird ein Reduktionsmittel verwendet, dessen Reduktionspotential $E_0$ <3,0 eV, insbesondere <0,8 eV beträgt. Diese Reduktionsmittel können dem Bleich(Wasch-)bad direkt zugegeben werden oder sie können bereits in entsprechenden Wasch-, Einweich-, Spül- oder Bleichmitteln zusammen mit dem Photobleichmittel und den üblichen Waschaktivsubstanzen und anderen Waschmittelbestandteilen enthalten sein. Der Zusatz der genannten Reduktionsmittel ist insbesondere dann vorteilhaft, wenn die Photobleiche im Einweichprozenss durchgeführt wird. Bevorzugte Reduktionsmittel, die im erfindungsgemässen Bleichverfahren in Frage kommen, sind nachstehend bei der Beschreibung der die erfindungsgemässen Photoaktivatoren enthaltenden Mittel beschrieben. Die vorliegende Erfindung betrifft auch photosensibilisierende (photoaktivierende) oder/und Singlett-Sauerstoff produzierende Mittel, die eine oder mehrere der in Formel (1) definierte Phthalocyaninverbindungen enthalten.

Die vorliegende Erfindung betrifft somit auch Mittel zur Durchführung des erfindungsgemässen Verfahrens, insbesondere antimikrobiell wirksame Mittel sowie Bleich-, Wasch-, Spül- und Einweichmittel. Diese Mittel sind dadurch gekennzeichnet, dass sie eine oder mehrere erfindungsgemässe Phthalocyaninverbindungen der Formel (1) enthalten. Die genannten Mittel können zusätzlich, je nach Anwendungsart, auch übliche Formulierungsbestandteile enthalten.

Bevorzugte derartige Mittel enthalten ein oder mehrere erfindungsgemässe Phthalocyaninverbindungen, ein oder mehrere anorganische Salze, wie z. B. NaCl, KCl, NaBr, KBr, $K_2SO_4$, $Na_2SO_4$, $K_2CO_3$, $Na_2CO_3$, $NaHCO_3$ u. a., insbesondere NaCl oder/und $Na_2SO_4$ und gegebenenfalls Wasser. Beispielsweise besteht ein derartiges Mittel aus etwa 1-80 % einer Verbindung der Formel (1), 1-40 % NaCl und/oder $Na_2SO_4$ und 0 bis 95 % Wasser. Diese Mittel können also in fester Form (z. B. Granulate) oder auch als wässrige Lösung vorliegen, z. B. in Form einer 5-50 %-igen, z. B. 5-20 %-igen Lösung.

Erfindungsgemässe Wasch-, Einweich- und Spülmittel mit Bleichwirkung enthalten zusätzlich zum Phthalocyaninwirkstoff z. B. übliche Waschmittelbestandteile, beispielsweise ein oder mehrere organische Detergentien, gegebenenfalls alkalische Gerüststoffsalze und gegebenenfalls weitere Bleichmittel, z. B. Perverbindungen wie etwa ein Perborat, Percarbonat usw.

Die erfindungsgemässen Waschmittel bzw. Einweichmittel enthalten z. B. die bekannten Mischungen von Waschaktivsubstanzen wie beispielsweise Seife in Form von Schnitzeln und Pulver, Synthetika, lösliche Salze von Sulfonsäurehalbestern höherer Fettalkohole, höher und/oder mehrfach alkylsubstituierter Arylsulfonsäuren, Sulfocarbonsäureester mittlerer bis höherer Alkohole, Fettsäureacylaminoalkyl- oder aminoarylglycerinsulfonate, Phosphorsäureester von Fettalkoholen usw. Als Additive, Komplexbildner, Bleichmittel usw. kommen z. B. Alkalisalze der Carboxymethylcellulose und andere "Soilredepositionsinhibitoren", ferner Alkalisilikate, Alkalicarbonate, Alkaliborate, Alkaliperborate, Alkalipercarbonate, Nitrilotriessigsäure, Aethylendiaminotetraessigsäure, Schaumstabilisatoren wie Alkanolamide höherer Fettsäuren, in Betracht. Ferner können in den Waschmitteln beispielsweise enthalten sein: antistatische Mittel, rückfettende Hautschutzmittel wie Lanolin, Enzyme, Antimikrobika, Parfüme und optische Aufheller.

Die erfindungsgemässen Waschmittel bzw. Einweichmittel enthalten die Phthalocyaninverbindungen der Formel (1) vorzugsweise in einer Menge von 0,0005 bis 1,5, insbesondere 0,005-1 Gewichtsprozent, bezogen auf das Gesamtwasch- oder -einweichmittel.

Beispielsweise enthalten erfindungsgemässe Wasch- bzw. Einweichmittel mit Bleichwirkung 0,005-1 Gew.-% der genannten Phthalocyaninverbindungen, 10-50 Gew.-% einer anionischen, nichtionischen, semipolaren, ampholytischen und/oder zwitterionischen oberflächenaktiven Substanz, 0-80 % eines alkalischen Gerüststoffsalzes und gegebenenfalls weitere übliche Waschmittelbestandteile, beispielsweise solche, die vorstehend erwähnt sind.

Als oberflächenaktive Substanzen in besagten Mitteln kommen beispielsweise auch wasserlösliche Alkybenzolsulfonate, Alkylsulfate, äthoxylierte Alkyläthersulfate, Paraffinsulfonate, α-Olefinsulfonate, α-Sulfocarbonsäuren, deren Salze und Ester, Alkylglyceryläthersulfonate, Fettsäuremonoglyceridsulfate oder -sulfonate, Alkylphenolpolyäthoxy-äthersulfate, 2-Acyloxyalkansulfonate, β-Alkyloxyalkansulfonate, Seifen, äthoxylierte Fettalkohole, Alkylphenole, Polypropoxyglykole, Polypropoxy-äthylendiamine, Aminoxide, Phosphinoxide, Sulfoxide, aliphatische sekundäre und tertiäre Amine, aliphatische quaternäre Ammonium-, Phosphonium- und Sulfoniumverbindungen oder Mischungen der genannten Substanzen in Betracht.

Beispiele für alkalische Gerüststoffsalze, die etwa in einer Menge von 10-60 Gew.-% in den erfindungsgemässen Mitteln vorhanden sein können, sind unter anderen:

wasserlösliche Alkalimetallcarbonate, -borate, -phosphate, -polyphosphate, -bicarbonate und -silicate, wasserlösliche Aminopolycarboxylate, Phytate, Polyphosphonate und -carboxylate, sowie wasserunlösliche Aluminiumsilicate.

Die erfindungsgemässen Waschmittel bzw. Bleichmittel können, wie bereits erwähnt, auch optische Aufheller enthalten. Als derartige Aufheller kommen alle in der Waschmittelindustrie üblichen Aufheller in Betracht. Besonders bevorzugt werden hingegen in erfindungsgemässen Wasch- bzw. Bleichmitteln optische Aufheller aus den Klassen der Distyrylbiphenylsulfonsäuren und deren Salzen oder/und 4,4'-Bis-(1, 2,3-triazol-2-yl)-2,2'-stilbendisulfonsäuren und deren Salzen und Mischungen davon eingesetzt. Falls erfindungsgemässe Mittel solche Aufheller enthalten, sind letztere vorzugsweise in einer Menge von 0,005-1,5 %, insbesondere von 0,01-0,5 %, bezogen auf das Gesamtgewicht des Mittels, in letzterem vorhanden. Als optische Aufheller können insbesondere solche der Formel

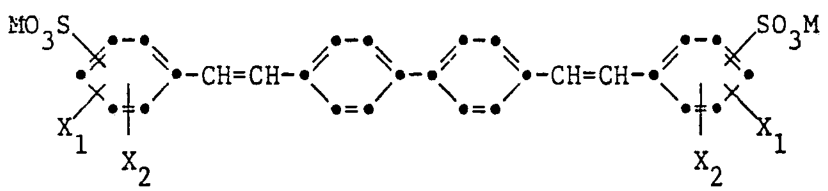

, (A1)

worin $X_1$ Wasserstof, Chlor, Brom oder Alkyl oder Alkoxy mit jeweils 1 bis 4 C-Atomen, $X_2$ Wasserstoff oder Alkyl mit 1 bis 4 C-Atomen und M Wasserstoff, ein Alkalimetall-, Ammonium- oder Aminsalzion bedeuten, vor allem der Formel

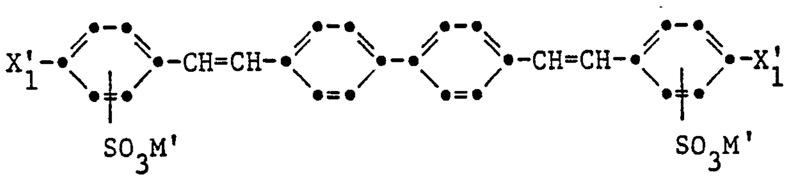

(A2)

worin $X'_1$ Wasserstoff oder Chlor und M' Wasserstoff, Natrium, Kalium oder Ammonium bedeuten, vorzugsweise der Formel

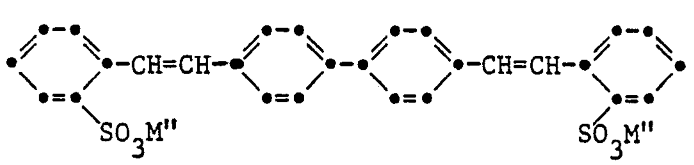

, (A3)

worin M'' Wasserstoff, Natrium oder Kalium bedeutet, oder/und der Formel

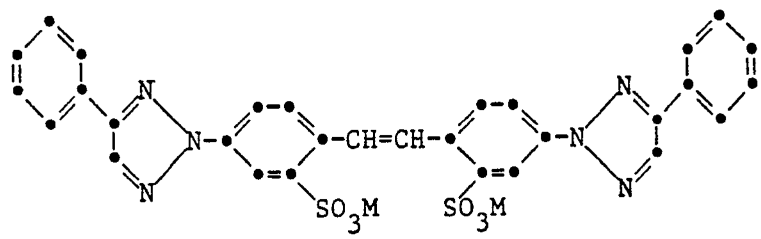, (A4)

worin M Wasserstoff, ein Alkalimetall-, Ammonium- oder Aminsalzion bedeutet, eingesetzt werden.

Die erfindungsgemässen antimikrobiell wirksamen Waschmittel enthalten die erfindungsgemässen Phthalocyaninverbindungen vorzugsweise in einer Menge von 0,0005 bis 1,5, insbesondere 0,005 bis 1 Gewichtsprozent, bezogen auf das Gesamtwaschmittel.

Im übrigen können erfindungsgemässe Waschmittel mit antimikrobieller Wirkung in gleicher Weise zusammengesetzt sein wie weiter oben für die erfindungsgemässen Wasch- und Einweichmittel mit Bleichwirkung beschrieben.

In den vorstehend beschriebenen Wasch-Bleich- und Einweichmitteln, die als Waschaktivsubstanzen insbesondere anionische, nichtionische, semipolare, ampholytische oder/und zwitterionische oberflächenaktive Substanzen enthalten, ist die Verwendung von solchen Phthalocyaninverbindungen der Formel (1) bevorzugt, die anionische oder nichtionische, vorzugsweise anionische wasserlöslichmachende Gruppen enthalten, obwohl auch die entsprechenden kationischen Verbindungen gute Bleicheffekte ergeben. Besonders bevorzugt sind dabei solche Verbindungen der Formel (1), in denen A $SO_3^{\ominus}$, $COO^{\ominus}$, $SO_2^{\ominus}$, $SO_2^{\ominus}NCN$ oder/und $SO_2^{\ominus}N\text{-}SO_2\text{-}R_6$, insbesondere $SO_3^{\ominus}$ bedeutet.

Die mit kationischen Gruppen A substituierten Phthalocyaninverbindungen der Formel (1) werden vorzugsweise als Photobleichmittel für Textilien in Wasch-, Einweich-, Spül- oder Nachbehandlungsmitteln eingesetzt, die kationische Textilbehandlungsmittel, z. B. kationische Tenside, Weichmacher, Antistatika, optische Aufheller und/oder Antimikrobika enthalten. In derartigen Mitteln kommt die Wirkung der kationischen Phthalocyanine der Formel (1) besonders gut zur Geltung.

Beispiele für kationische Textilbehandlungsmittel (z. B. Tenside, Weichmacher, Antistatika, optische Aufheller), die in den erfindungsgemässen Mitteln vorhanden sein können, sind in der EP-A-35 470 von Seite 10, 2. Absatz, bis Seite 16, 2. Absatz angegeben. Diese Beschreibungsteile werden hiermit zu einem Teil der vorliegenden Anmeldung erklärt. In der genannten EP-A sind auch im Beispielteil Zusammensetzungen derartiger kationischer Textilbehandlungsmittel beschrieben. Die erfindungsgemässen kationischen Phthalocyanine der Formel (1) können in analoger Weise in derartige Mittel inkorporiert werden. Solche Mittel, insbesondere Textilnachbehandlungsmittel und Spülmittel, sind in der Regel flüssig. Die darin enthaltenen Wirkstoffe werden vorzugsweise in Wasser emulgiert. Neben den genannten Wirkstoffen können die erfindungsgemässen Textilbehandlungsmittel (Spül-, Nachbehandlungs-, Waschmittel) auch noch andere fakultative Bestandteile enthalten. Beispiele für solche Bestandteile sind ebenfalls in der EP-A-35 470 von Seite 16, letzter Absatz, bis Seite 22, erster Absatz, beschrieben. Diese Beschreibung gilt hiermit ebenfalls als in die vorliegende Anmeldung aufgenommen.

Bevorzugte kationische Textilbehandlungsmittel, nämlich insbesondere Weichmacher, Antistatika bzw. Tenside, in den vorstehend beschriebenen Mitteln enthaltend kationische Verbindungen der Formel (1), sind die folgenden:

1) Quaternäre Ammoniumsalze der Formel

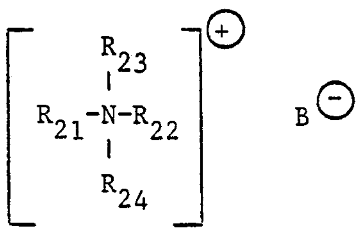 ,

worin $R_{21}$ Wasserstoff oder eine aliphatische Gruppe mit 1 bis 22 C-Atomen, $R_{22}$ eine aliphatische Gruppe mit 10 bis 22 C-Atomen, $R_{23}$ und $R_{24}$ unabhängig voneinander Alkyl mit 1 bis 4 C-Atomen und $B^{\ominus}$ ein Anion bedeuten.

Beispiele für quaternäre Ammonium-Weichmacher sind:
Tallyltrimethylammoniumchlorid, Ditallyldimethylammoniumchlorid; Ditallyldimethylammoniumsulfat, Dihexadecyldimethylammoniumchlorid, Dioctadecyldimethylammoniumchlorid, Dieicosyldimethylammoniumchlorid, Didocosyldimethylammoniumchlorid, Dihexadecyldiäthylammoniumchlorid, Dihexadecyldimethylammoniumacetat, Ditallyldipropylammoniumphosphat, Ditallyldimethylammoniumnitrat, Dicocoyldimethylammoniumchlorid.

2) Quaternäre Imidazoliniumsalze der Formel

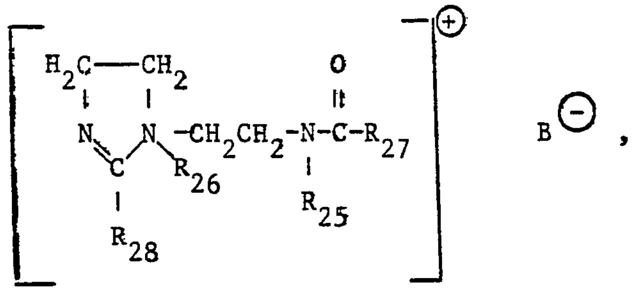

worin $R_{25}$ Wasserstoff oder Alkyl mit 1 bis 4 C-Atomen, $R_{26}$ Alkyl mit 1 bis 4 C-Atomen, $R_{27}$ Alkyl mit 1 bis 22 C-Atomen, $R_{28}$ Wasserstoff oder Alkyl mit 1 bis 22, vorzugsweise 15-22 C-Atomen und $B^{\ominus}$ ein Anion bedeuten. Bevorzugt steht $R_{27}$ und gegebenenfalls auch $R_{28}$ jeweils für Alkyl mit 12 bis 22 C-Atomen.

Beispiele für bevorzugte Imidazoliniumverbindungen der vorstehenden Formel sind: 1-Methyl-1-stearoylamidoäthyl-2-heptadecyl-4,5-dihydro-imidazoliniummethosulfat, 1-Methyl-1-palmitoylamidoäthyl-2-octadecyl-4,5-dihydroimidazoliniumchlorid, 2-Tallyl-1-methyl-1-talloylamido-äthyl-4,5-dihydro-imidazolinium-methosulfat.

Alle vorstehend beschriebenen erfindungsgemässen Wasch-, Spül-, Einweich- und Nachbehandlungsmittel mit Bleichwirkung, die ein oder mehrere Phthalocyanine der Formel (1) als Photobleichmittel enthalten, können gegebenenfalls zusätzlich ein Reduktionsmittel enthalten. Die Gegenwart eines solchen verbessert in manchen Fällen die Bleichwirkung des Photoaktivators je nach zu behandelndem Schmutz und Substrat. Die Anwendung erfolgt in üblicher Weise (Bestrahlung mit Licht). Besonders gute Ergebnisse werden mit dieser "reduktiven Bleiche" im Einweichprozess erzielt. Zu diesem Zweck werden die Textilien in einem Bad, das das Einweich(Wasch)mittel mit dem Photoaktivator und dem Reduktionsmittel enthält, eingeweicht und direkt mit Licht, vorzugsweise mit Sonnenlicht, bestrahlt.

Als Reduktionsmittel kommen Substanzen in Frage, wie sie in der EP-A-87 833 als "electron donors" definiert und beschrieben sind. Insbesondere sind solche Reduktionsmittel verwendbar, die ein Reduktionspotential von $<3{,}0$ eV, vor allem $<0{,}8$ eV aufweisen. Beispiele für derartige Reduktionsmittel (Elektronendonatoren) sind Alkalimetallsulfite, Cystein, Alkalimetallthiosulfate, Fe(II)-Salze wie $FeSO_4$, Sn(II)-Salze wie $SnCl_2$ usw. Bevorzugt sind davon Alkalimetallsulfite, insbesondere Natriumsulfit. Sofern ein vorstehend genanntes Reduktionsmittel (Elektronendonator) in erfindungsgemässen Wach, Bleich-, Einweich- bzw. Spülmitteln enthalten ist, beträgt seine Konzentration beispielsweise 1 bis 40 Gew.-%, bezogen auf das Gesamtmittel.

Die nachfolgenden Beispiele erläutern die Verfahren zur Herstellung der erfindungsgemässen Phthalocyaninverbindungen, deren Verwendung und sie enthaltende Mittel näher, ohne dass damit eine Beschränkung auf den Inhalt dieser Beispiele ausgedrückt wird. Teile- und Prozentangaben bedeuten in den Beispielen ebenso wie in der übrigen Beschreibung immer Gewichtsteile und Gewichtsprozent, sofern nichts anderes angegeben ist.

**Beispiel 1:**

Bei Raumtemperatur werden 30 Teile Germanium(IV)phthalocyanin langsam in 140 Vol.Teile Chlorsulfonsäure eingetragen. Nach einstündigem Rühren wird das Reaktionsgemisch auf 100 bis 105°C erwärmt. Nach 2 Stunden wird die Reaktionstemperatur auf 115 bis 120°C erhöht und weitere 2 Stunden gehalten. Die auf Raumtemperatur abgekühlte Reaktionsmasse wird auf Eis/Wasser ausgetragen, die entstandene Suspension abgenutscht und mit eiskalter, 5 %-iger Kochsalzlösung gewaschen. Das Nutschgut wird in 1000 Teilen Wasser verrührt und mit 10 Vol.Teilen Pyridin versetzt. Bei einer Temperatur von 20 bis 25°C wird durch Zugabe von Natronlauge der pH-Wert bei 9 bis 9,5 gehalten, bis letzterer nicht mehr absinkt. Die Lösung wird im Vakuum zur Trockne eingedampft. Man erhält 58 Teile eines in Wasser mit blauer Farbe löslichen Pulvers.

Die Analyse ergibt, dass das Phthalocyaninmolekül mit durchschnittlich 2,8 Sulfonsäuregruppen substituiert wurde. Das Produkt entspricht also der ungefähren Formel

$Ge(IV)Pc(SO_3Na)_{ca.2,8}$ (103),

worin Ge(IV)Pc der Rest des Ge(IV)-Phthalocyanins ist, und weist ein $\lambda_{max}$ von 680 nm auf (gemessen in Dimethylformamid/Wasser 1 : 1).

Das als Ausgangsmaterial benötigte Germanium(IV)phthalocyanin ist aus der Literatur bekannt. Siehe z. B. J. Amer. Chem. Soc. 82, 5790 (1960).

**Beispiel 1a:**

Bei Raumtemperatur werden 30 Teile Germanium(IV)phthalocyanin langsam in 140 Vol. Teile Chlorsulfonsäure eingetragen. Nach einstündigem Rühren wird das Reaktionsgemisch auf 100 bis 105°C erwärmt. Nach 2 Stunden wird die Reaktionstemperatur auf 115 bis 120°C erhöht und weitere 2 Stunden gehalten. Die auf Raumtemperatur abgekühlte Reaktionsmasse wird auf Eis/Wasser ausgetragen, die entstandene Suspension abgenutscht und mit eiskalter, 5 %-iger Kochsalzlösung gewaschen.

Die so erhaltene Sulfochloridpaste wird in 600 Teilen Eis/Wasser suspendiert, mit 11 Teilen Cyanamid versetzt und mit Natronlauge auf pH 10 gehalten. Das Reaktionsgemisch wird bei Raumtemperatur so lange gerührt, bis der pH-Wert ohne weitere Laugenzugabe konstant bleibt. Die entstandene Lösung wird zur Trockne eingedampft. Man erhält 70 Teile eines blauen Pulvers.

35 Teile des erhaltenen Pulvers werden in 500 Teilen Wasser gelöst, mit konz. Salzsäure auf pH 2 gestellt und zur Trockne eingedampft. Der Rückstand wird fein pulverisiert und dann in 250 Volumenteilen 1N Salzsäure verrührt.

Die Suspension wird filtriert und der Rückstand mit 250 Volumenteile 1N Salzsäure gewaschen. Das Filtergut wird in 400 Teilen Wasser angerührt, mit Natronlauge auf pH 7 gestellt und anschliessend eingedampft. Man erhält 21 Teile eines blauen Pulvers.

Die so erhaltene Verbindung entspricht der Formel

$$Ge(IV)Pc\text{-}[\text{-}SO_2\text{-}N^{\ominus}\text{-}CN\ Na^{\oplus}]_{ca.\ 2.8}$$

$(\lambda_{max} = 680\ nm)$

**Beispiel 2:**

Ein 1 g schweres, mit Tee angeschmutztes Baumwollgewebe *) wird bei 40°C unter Belichtung mit einer 250W IR-Lampe **) eine Stunde lang unter Rühren in 100 ml einer wässrigen Waschflotte behandelt, die 0,005 %, bezogen auf das Gewicht des Gewebes, der Verbindung der Formel (103) und 0,5 g eines Waschmittels der folgenden Zusammensetzung enthält:

| | |
|---|---|
| Natriumdodecylbenzolsulfonat | 16 % |
| Natriumtripolyphosphat | 43 % |
| Natriumsilikat | 4 % |
| Magnesiumsilikat | 2 % |
| Fettalkoholsulfat | 4 % |
| Natriumcarboxymethylcellulose | 1 % |
| Natriumsalz der Aethylendiamintetraessigsäure | 0,5 % |
| Natriumsulfat | 29,5 % |

Anschliessend wird das Gewebestück gespült und getrocknet und danach visuell beurteilt, wobei sich zeigt, dass seine Helligkeit weit über jener des angeschmutzten Gewebes liegt.

Der Bleichgrad des behandelten Gewebestückes wird auch durch Messung des Weissgrades (Helligkeitswertes) Y (ausgedrückt in %, bezogen auf das Absolutweiss gemäss CIE-Empfehlung vom 1.1.1969) mit Hilfe eines ®Elrepho-Spektrophotometers der Firma ZEISS ermittelt. Die gemessenen Werte bestätigen den visuellen Eindruck und zeigen, dass durch den Zusatz des Photosensibilisators der Formel (103) ein beträchtlicher Helligkeitsgewinn (ΔY) gegenüber dem ohne Photosensibilisator gewaschenen Vergleichsgewebe erzielt wird.

*) Die Anschmutzung des Baumwollgewebes mit Tee wird folgendermassen durchgeführt:

15 g Tee ("Fine Ceylon Fannings Tea") werden in 600 ml entsalztem Wasser 1 Stunde lang gekocht und anschliessend filtriert. Die filtrierten Teeblätter werden mit 400 ml entsalztem Wasser aufgenommen und erneut ca. 60 Minuten gekocht. Die beiden Filtrate werden vereint und mit entsalztem Wasser auf 1000 ml aufgefüllt. In diesem Tee werden 45 g Baumwollgewebe (gebleicht und laugiert) unter stetiger Bewegung bei 100°C während 2 1/2 Stunden behandelt; sodann "färbt" man auf abkühlendem Bade weitere 16 Stunden. Danach wird die Teeflotte mit 5 g Kochsalz versetzt und nochmals 2 1/2 Stunden bei 100°C behandelt. Schliesslich wird abgekühlt und die angeschmutzte Baumwolle zweimal bei 60°C gespült und bei 100°C getrocknet. Abschliessend wird das angeschmutzte Gewebe noch mit einer Flotte, die 5 g/l Waschmittel (Zusammensetzung siehe oben) enthält, bei 90°C 20 Minuten und einem Flottenverhältnis von 1 : 20 gewaschen, warm und kalt gespült und bei 100°C in einem Umluftofen getrocknet.

**) Verwendete Lampe: "Philips"-Infrarot-Lampe (weiss) 220/230V, 250W, mit Reflektor Typ 13372 E/06. Die Lampe ist ca. 25 cm über der Waschflotte angebracht.

**Beispiel 3:**

Man verfährt wie in Beispiel 2 beschrieben, setzt jedoch an Stelle des mit Tee angeschmutzten Testgewebes ein mit Rotwein (EMPA-Testgewebe Nr. 114, siehe Beispiel 9), Heidelbeersaft oder Kirschensaft angeschmutztes Gewebe ein. Auch diese Testgewebe werden durch den Photoaktivator der Formel (103) ausgezeichnet gebleicht und es wird ebenfalls ein beträchtlicher Helligkeitsgewinn gegenüber dem ohne Photoaktivator gewaschenen Vergleichsgewebe erzielt.

**Beispiel 4:**

Jeweils 5 Muster à 5 g eines mit einem braunen Farbstoff*) gefärbten Baumwollgewebes werden in je 500 ml einer Waschflotte gegeben, die 5 g/l eines Waschmittels der in Beispiel 2 angegebenen Zusammensetzung sowie 0,005 %, bezogen auf das Gewebegewicht , der Verbindung der Formel (103) enthält. Unter ständiger Bewegung der zu bleichenden Muster werden diese bei 50°C während 120 Minuten und unter Belichtung mit einer in Beispiel 6 beschriebenen Lampe gewaschen. Die Muster werden danach gespült, getrocknet und anschliessend wird der Bleichgrad der getrockneten Muster mit Hilfe eines ®Elrepho-Photometers der Fa. ZEISS (Normlichtart D 65, 2 Grad Normalbeobachter, Messblende 35 mm Ø) in Form von Helligkeitswerten, ausgedrückt in %, bezogen auf das Absolutweiss gemäss CIE-Empfehlung von 1.1. 1969, gemessen. Die gemessenen Helligkeitswerte, die weit über jenen des angefärbten Gewebes vor und nach der Wäsche ohne Photoaktivator liegen, zeigen, dass das angeschmutzte Gewebe mit Hilfe des eingesetzten Photoaktivators hervorragend gebleicht wird.

*) Die Anfärbung der Baumwollmuster wird folgendermassen durchgeführt:

150 mg des im Handel erhältlichen braunen Farbstoffes der Formel

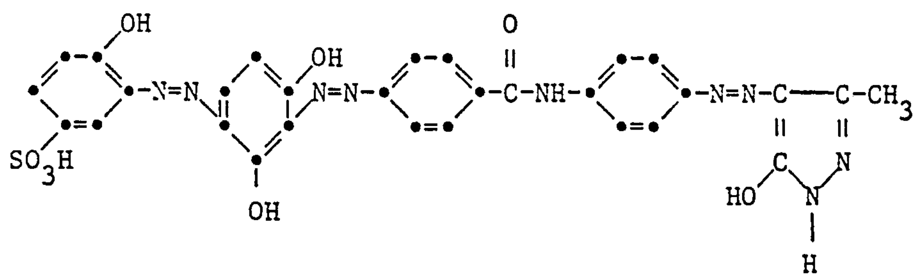

werden in 2000 ml Wasser, die 1 g Soda enthalten, bei einer Temperatur von 50°C gelöst. In dieser Farbflotte werden 100 g Baumwollgewebe (gebleicht, laugiert) unter ständiger Bewegung gefärbt, indem das Bad während 30 Minuten auf 90°C erhitzt wird. Bei 90°C färbt man 90 Minuten, wobei in dieser Zeitspanne 20 g Glaubersalz in 4 gleichgrossen Portionen in Abständen von 15 Minuten zugesetzt werden.

Nach dem Färben wird 2mal kalt gespült und in einem Bad, das 0,75 g/l Kupfersulfat kristallisiert und 1 ml/l Eisessig enthält, bei 60°C und einem Flottenverhältnis von 1 : 20 20 Minuten lang gekupfert. Anschliessend wird die Färbung 2mal kalt gespült und in einem Heissluftofen bei 100°C getrocknet.

**Beispiel 5:**

Jeweils 10 g eines mit Rotwein angeschmutzten Testgewebes (EMPA-Testgewebe Nr. 114, erhältlich bei der Eidgenössischen Materialprüf- und Versuchsanstalt, CH-9001 St. Gallen, Unterstrasse 11) werden bei einem Flottenverhältnis von 1 : 50 bei 50°C während 30 Minuten in Waschflotten gewaschen, die folgende Bestandteile enthalten:

Flotte 1: 4 g/l des Waschmittels der in Beispiel 2 angegebenen Zusammensetzung

Flotte 2: 4 g/l des Waschmittels der in Beispiel 2 angegebenen Zusammensetzung
0,0005 g der Verbindung der Formel (103)

Flotte 3: 4 g/l des Waschmittels der in Beispiel 2 angegebenen Zusammensetzung
1 g/l Natriumperborat
0,5 g/l Tetraacetyläthylendiamin (Bleichaktivator)

Flotte 4: 4 g/l des Waschmittels der in Beispiel 2 angegebenen Zusammensetzung
1 g/l Natriumperborat
0,5 g/l Tetraacetyläthylendiamin
0,0005 g der Verbindung der Formel (103).

Nach dem Waschen werden die Gewebestücke kurz gespült und dann 2 Stunden lang an die Sonne gelegt und mehrmals befeuchtet. Danach wird der Bleichgrad (die Helligkeit) der Gewebemuster bestimmt, wie in Beispiel 2 bzw. 4 angegeben.

Die erhaltenen Ergebnisse zeigen, dass die in der Flotte 2 gewaschenen Gewebemuster wesentlich höhere Helligkeitswerte aufweisen als jene, die in Flotte 1 gewaschen werden. Der Vergleich der Waschversuche in den Flotten 3 und 4 zeigt, dass der Zusatz eines Photoaktivators (hier der Formel (103)) zu einer aktivierten Perboratbleichflotte die Helligkeit der gewaschenen Gewebestücke zusätzlich noch beträchtlich zu steigern vermag. Die in der Flotte 4 gewaschenen Gewebeproben sind deutlich heller als jene, die in Flotte 3 gewaschen werden.

**Beispiel 6:**

Ein Waschmittelslurry, bestehend aus 50 Teilen deionisiertem Wasser und 50 Teilen eines Waschmittels folgender Zusammensetzung wird hergestellt:

8,0 % Lineares Natrium-alkylbenzolsulfonat (Kettenlänge des Alkylesters: $\bar{C}_{11.5}$).
2,9 % Talfalkohol-tetradekan-äthylenglykoläther (14 ÄO)
3,5 % Natriumseife (Kettenlängen $C_{12-16}$: 13-26 %, $C_{18}$-$C_{22}$: 74-87 %)
43,8 Natriumtriphosphat
7,5 % Natriumsilikat ($SiO_2$:$Na_2O$ = 3,3 : 1)
1,9 % Magnesiumsilikat
1,2 % Carboxymethylcellulose
0,2 % Na-Äthylendiamintetraacetat
21,2 % Natriumsulfat
0,03 % Photoaktivator der Formel (103)
0,13 % 4,4'-Bis-(2-sulfostyryl)biphenyl, Na-Salz (optischer Aufheller)
ad 100 % Wasser.

Photoaktivator und Aufheller werden dem oben definierten, diese beiden Komponenten noch nicht enthaltenden Waschmittelslurry unter weitgehendem Lichtabschluss zugegeben und im Trockenkasten bei einem Vakuum von ca. 400 Torr und 80° C während 4 Stunden getrocknet. Die erhaltenen Waschmittelkrusten werden durch ein Sieb gepresst, unter welchem sich ein anderes Sieb befindet, so dass ein gleichkörniges Waschpulver erzeugt wird.

Als Testsubstrate werden Lappen aus gebleichtem Baumwollgewebe verwendet, die durch Fruchtsäfte (Kirschen-, Holunder-, Brombeer-, Johannisbeer- und Heidelbeersaft), Tee (siehe Beispiel 2), Blut (EMPA-Testgewebe, Art 103, Serie 23) oder Rotwein (EMPA-Testgewebe Nr. 114) angeschmutzt sind.

Testseifen aus den eben beschriebenen angeschmutzten Geweben werden je in einer Flotte, enthaltend 4 g pro Liter des oben definierten Waschmittels, bei einem Flottenverhältnis von 1 : 20 während 30 Minuten bei 50° C gewaschen, dann kurz gespült und schleuderfeucht am Tageslicht an einer Leine aufgehängt und während 6 Stunden trocknen gelassen, wobei alle 40 Minuten mit einer alkalischen Lösung von pH 9 besprüht wird. Diese Waschversuche werden auch mit einem Waschmittel durchgeführt, das keinen optischen Aufheller oder keinen Photoaktivator oder beide nicht enthält. Der Grad der Schmutzentfernung wird visuell beurteilt. Bei Verwendung des Waschmittels ohne Aufheller und Photoaktivator wird eine mehr oder weniger bescheidene Schmutzentfernung erreicht. Bei Anwesenheit des Photoaktivators (ohne Aufheller) werden sehr gute und deutliche Bleicheffekte erzielt. Bei Anwesenheit des Aufhellers (ohne Photoaktivator) erhält man Bleicheffekte,

die aber geringer sind als bei Anwesenheit des Photoaktivators allein. Bei Anwesenheit von Photoaktivator und Aufheller werden die weitaus besten Resultate bei allen Anschmutzungsarten erzielt. Es resultiert ein jeweils ausserordentlich helles, stark gebleichtes Baumwollgewebe.

An einem standardisierten braungefärbten Baumwollstück (siehe Beispiel 4) werden die erhaltenen Effekte farbmetrisch ausgewertet. Die visuell erhaltenen Befunde werden dabei voll bestätigt.

Völlig analoge Resultate erhält man, wenn man im Waschmittel der oben beschriebenen Zusammensetzung als optischen Aufheller 4,4'-Bis-(4-phenyl-1,2,3-triazol-2-yl)-2,2'-stilbendisulfonsäure (K-Salz) einsetzt.

**Beispiel 7:**

100 Teile eines Grundwaschmittels der in Beispiel 2 angegebenen Zusammensetzung werden mit 50 ml Wasser angeschlämmt. 0,02 Teile des Photoaktivators der Formel (103) und 0,15 Teile 4,4'-Bis-(2-sulfostyryl)biphenyl, Na-Salz (optischer Aufheller) werden in wenig Wasser gelöst, der Waschmittelaufschlämmung zugesetzt und gut vermischt. Die Aufschlämmung wird bei 105°C in einem Trockenschrank getrocknet, pulverisiert und wie in Beispiel 6 beschrieben zu einem gleichkörnigen Waschpulver verarbeitet.

Ein 5 g schweres, mit einem braunen Farbstoff gemäss Beispiel 4 gefärbtes Baumwollgewebe wird bei 35°C 30 Minuten lang in 100 ml einer Waschflotte behandelt, die 0,5 g des Waschpulvers enthält, das wie vorstehend beschrieben erhalten wurde. Ohne zu spülen wird das Gewebe dann auf mit der Waschflotte getränktes Filterpapier gelegt und mit einer 250 W IR-Lampe (wie in Beispiel 2 beschrieben; Abstand der Lampe zum Gewebe: ca. 30 cm) während 90 Minuten bestrahlt. Danach wird gespült, getrocknet und der Bleichgrad (Helligkeitszuwachs) wie in Beispiel 2 beschrieben bestimmt. Der gesamte Vorgang (Waschen, Belichten, Helligkeitsmessung) wird fünfmal wiederholt. Ergebnis: Bis zum 5. Zyklus nimmt der Helligkeitswert des Gewebes kontinuierlich zu, ohne dass eine Vergrünungstendenz desselben festzustellen wäre.

**Beispiel 8:**

Prüfung der Aktivität gegen Bakterien

Methode: Einer wässrigen Lösung, die eine der Verbindungen der Formeln (103) in einer Konzentration von 0,01, 0,1 und 1,0 ppm enthält, wird eine Keimsuspension von Staphylococcus aureus ATCC 6538 mit einer definierten Keimmenge pro ml zugegeben. Diese Testsuspens ion befindet sich in einem Becherglas unter einer wassergekühlten Glasplatte, um Erwärmung durch die nachfolgende Belichtung zu vermeiden. Es wird nun mit einer Glühlampe oder einer Infrarotlampe (Infrarotlampe "Weiss", Philips IR, 250 W, Typ 13372 E/06), die sich in einem Abstand von 20 cm über der Suspensionsoberfläche befindet, 5, 10, 20, 30 bzw. 60 Minuten lang bestrahlt. Danach wird in üblicher Weise die Keimzahl durch Parallelzählungen bestimmt. Die jeweilige Keimreduktion wird in Zehnerpotenzen nach der Formel $\bar{x} = -\log_{10} \frac{N}{N_0}$ berechnet, wobei $N_0$ die Keimeinsaat und N die Zahl der überlebenden Keime ist.

Die Ergebnisse zeigen, dass die geprüften Verbindungen die Zahl der Testkeime, je nach eingesetzter Konzentration und Belichtungsdauer, um 2 bis 6 Zehnerpotenzen reduzieren.

**Beispiel 9:**

Prüfung der Desinfektionswirkung auf Textilien: Ein Stück Baumwollgewebe wird auf einem Metallrechen aufgespannt und mit einer im Beispiel 13 beschriebenen Prüfsuspension (enthaltend eine der Verbindungen der Formeln (103) und einen Testkeimstamm) beimpft. Der Metallrechen, der an einen Motor angeschlossen ist, wird nun gedreht und mit einer Infrarotlampe bestrahlt. Zwischen Lampe und Gewebestück ist eine Glasplatte angebracht, die mit fliessendem Wasser gekühlt wird, um eine Erwärmung des Gewebestückes zu vermeiden. Unter gleichen Versuchsbedingungen wird parallel dazu ein Gewebestück behandelt, auf das jedoch keine antimikrobielle Wirksubstanz aufgebracht worden war. Nach 1-stündiger Belichtung werden die Keimzahlen quantitativ ermittelt und die durch das jeweilige Phthalocyanin bewirkte Keimreduktion festgestellt. Getestet wird die Wirkung gegenüber Staphylococcus aureus ATCC 6538. Es werden ähnliche Keimreduktionen festgestellt wie in Beispiel 9.

**Beispiel 10:** Oberflächendesinfektion

Emaillierte Fliesen der Grösse 4 x 4 cm werden mit einer Staphylococcus aureus ATCC 6538-Keimsuspension beimpft; ca. $10^5$ Keime werden dabei gleichmässig auf die Oberfläche einer Fliese verteilt. Dann wird auf die

Oberfläche eine 1 ppm einer der Verbindungen der Formeln (103) enthaltende wässrige Lösung aufgesprüht. Die Oberfläche wird dann mit einer Glühlampe (250 W, Abstand: 20 cm) 30 bzw. 45 Minuten bestrahlt. Nach dieser Zeit erfolgt die Probeentnahme durch Abklatschen in Rodac-Schalen. Nach 45 Minuten ist bei Behandlung mit den genannten Verbindungen kein Wachstum des Keimes mehr zu beobachten.

**Beispiel 11:** Entkeimung eines Kläranlage-Effluenten

Es wird eine Schlammprobe aus einer Laborkläranlage entnommen und durch ein Papierfilter filtriert. Zu dem Filtrat, das ca. $10^6$ Keime/ml enthält, wird jeweils eine der zu prüfenden Phthalocyaninverbindungen der Formeln (103) zugegeben, bis deren Konzentration im Filtrat 1 ppm beträgt. Danach wird letzteres mit Standardlicht 380-730 nm, 300 mW/$cm^2$, beleuchtet. Nach verschiedenen Zeitabständen wird die verbliebene Keimzahl bestimmt. Nach 45 Minuten sind bereits keine Staphylokokken mehr vorhanden. Nach längerer Belichtungsdauer (1-mehrere Stunden) nimmt auch die Zahl der übrigen im Filtrat vorhandenen Keime deutlich ab.

**Beispiel 12:** Entkeimung von Schwimmbecken

Im Freien werden Schwimmbecken mit je 5000 l Wasser Inhalt aufgestellt. Das Wasser eines Beckens wird mit jeweils einer der Verbindungen der Formeln (103) in einer Konzentration von 0,5 ppm versetzt. In Abständen von 1-5 Tagen werden Wasserproben entnommen und die Keimzahlen quantitativ bestimmt. In der mikrobiologischen Prüfung wird a) die Gesamtkeimzahl und b) die Zahl der coliformen Keime bstimmt.

**Ergebnis:**

Im Becken, das keine der geprüften Phthalocyaninverbindungen enthält, vermehren sich die coliformen Keime auf $2\text{-}3.10^1$ Keime/100 ml. Im eine Wirksubstanz enthaltenden Becken werden bis zum 16. Versuchstag keine coliformen Keime festgestellt.

Für einen weiteren Test wird am 16. Versuchstag dem Wasser eine Keimsuspension enthaltend Staphylococcus aureus ATCC 6538 und Escherichia coli ATCC 11229 in einer Menge von je 50 Keimen pro 100 ml Beckeninhalt zugegeben. Eine Messung ergibt sofort nach dem Keimeinsatz eine gleichmässige Verteilung im Becken. Nach 24 Stunden werden im die Wirksubstanz enthaltenden Becken keine coliformen Keime sowie keine Staphylokokken nachgewiesen (Entnahme jeweils 100 ml Wasser). Die Gesamtkeimzahl, bestehend aus autochthoner (schwimmbeckeneigener) Keimflora blieb während der Versuchsdauer konstant.

**Patentansprüche**

1. Phthalocyaninverbindungen der Formel

$$\left[\mathrm{MePc}\right]\begin{matrix}(AZ)_n\\(Y)_p\end{matrix}$$

worin

MePc für das Ge(IV)-Phthalocyanin- oder Naphthalocyaninringsystem steht,
A eine anionische, nichtionische oder kationische wasserlöslichmachende Gruppe,
Z das Gegenion zu A, falls A eine ionische Gruppe ist und
Z ist Null, falls
A eine nichtionische Gruppe ist,
n eine beliebige Zahl von 1 bis 8,
Y einen neutralen, nicht wasserlöslichmachenden Substituenten und
p eine beliebige Zahl von 0 bis 6 bedeuten, wobei die im Molekül vorhandenen Gruppen AZ und Y jeweils gleich oder verschieden sein können, und die Summe n + p höchstens 8 beträgt.

2. Phthalocyaninverbindungen nach Anspruch 1, worin A eine anionische Gruppe und Z ein Kation ist.

3. Phthalocyaninverbindungen nach Anspruch 2, worin A eine Sulfo-, Carboxyl-, Phosphat-, Sulfat-, Sulfinyl-, Disulfimid- oder Cyanimidgruppe oder ein eine oder mehrere der vorgenannten Gruppen enthaltender Rest ist.

4. Phthalocyaninverbindungen nach Anspruch 3, worin A eine der folgenden Gruppen bedeutet:

$-SO_3^{\ominus}$, $-COO^{\ominus}$, $-SO_2^{\ominus}$, $-SO_2-Alk-OSO_3^{\ominus}$, $-SO_2-Alk-SO_3^{\ominus}$,

$-Y_4-\underset{R_4}{\underset{|}{N}}-Alk-OSO_3^{\ominus}$, $-Y_4-\underset{R_4}{\underset{|}{N}}-Alk-SO_3^{\ominus}$, $-(CH_2CH_2O)_m-SO_3^{\ominus}$,

$-(CH_2CH_2O)_m-COO^{\ominus}$,

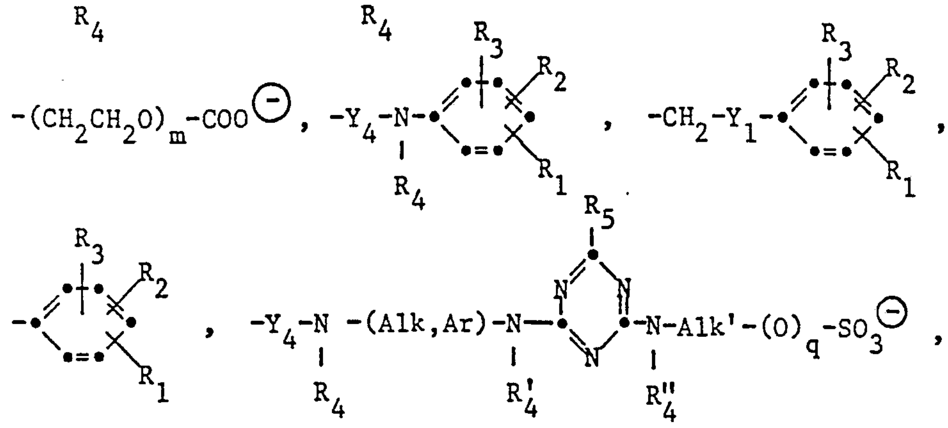

$-SO_2\overset{\ominus}{N}-CN$ oder $-SO_2\overset{\ominus}{N}-SO_2-R_6$,

worin $R_1$, $R_2$ und $R_3$ unabhängig voneinander Wasserstoff, Alkyl, Hydroxy, $SO_3^{\ominus}$ oder $COO^{\ominus}$, wobei mindestens einer dieser Substituenten für $SO_3^{\ominus}$ oder $COO^{\ominus}$ steht $R_4$ $R'_4$ und $R''_4$ unabhängig voneinander gegebenenfalls substituiertes Alkyl oder Wasserstoff, $R_5$ Wasserstoff oder Halogen, $R_6$ gegebenenfalls substituiertes Alkyl, gegebenenfalls substituiertes Phenyl oder Naphthyl, Ar einen gegebenenfalls substituierten Phenyl- oder Naphthylrest, Alk und Alk' jeweils eine gegebenenfalls substituierte Alkylengruppe, $Y_1$ $N-R_4$ oder S, $Y_4$ $-SO_2-$ oder -CO-, vorzugsweise $-SO_2-$, m eine Zahl von 1 bis 30 und q 0 oder 1 bedeuten.

5. Phthalocyaninverbindungen nach Anspruch 4, worin A eine Gruppe der Formel $-SO_3^{\ominus}$, $-COO^{\ominus}$, $-SO_2^{\ominus}$, $-SO_2^{\ominus}N-CN$ oder $-SO_2^{\ominus}N-SO_2-R'_6$ bedeutet, worin $R'_6$ für $C_1$-$C_4$-Alkyl, Phenyl, ($C_1$-$C_4$-Alkyl)phenyl, Chlorphenyl oder Methoxyphenyl steht.

6. Phthalocyaninverbindungen nach Anspruch 5, worin A $SO_3^{\ominus}$ und Z ein Wasserstoff-, Alkalimetall- oder unsubstituiertes oder substituiertes Ammoniumion bedeuten.

7. Phthalocyaninverbindungen nach Anspruch 1, worin A eine kationische Gruppe und Z ein Anion ist.

8. Phthalocyaninverbindungen nach Anspruch 7, worin A eine quaternäre Ammoniumgruppe oder eine ternäre Sulfoniumgruppe oder ein eine oder mehrere der vorgenannten Gruppen enthaltender Rest ist.

9. Phthalocyaninverbindungen nach Anspruch 7, worin A für eine Gruppe der Formel

$-SO_2\underset{R_4}{\underset{|}{N}}-R_7-Y_2^{\oplus}$, $-CO\underset{R_4}{\underset{|}{N}}-R_7-Y_2^{\oplus}$, $-(CH_2)_q-Y_3^{\oplus}$ oder $-CH_2NHCOCH_2-Y_3^{\oplus}$

steht, worin q für 0 oder 1 steht, $R_7$ unverzweigtes oder verzweigtes Alkylen mit 1 bis 8 C-Atomen oder 1,3- oder 1,4-Phenylen, $R_4$ Wasserstoff oder gegebenenfalls substituiertes Alkyl, $Y_2^{\oplus}$ eine Gruppe der Formel

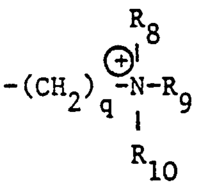 , 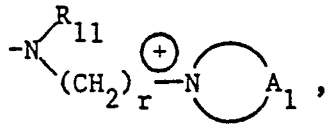 , 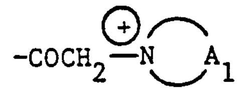 ,

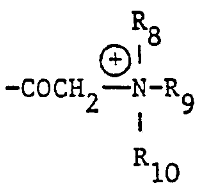

und für den Fall, dass $R_7$ = Alkylen, auch eine Gruppe der Formel

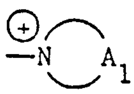 , 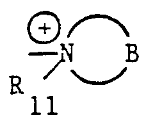 , 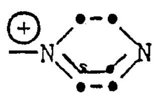 , 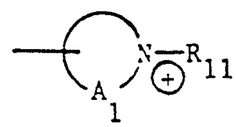 ,

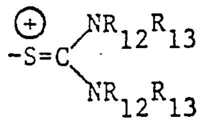 oder 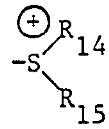 ,

$Y_3^{\oplus}$ eine Gruppe der Formel 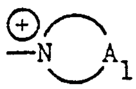 , 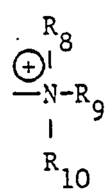 , 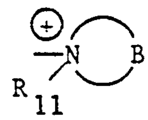 ,

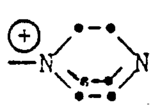 , 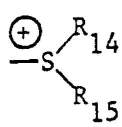 , 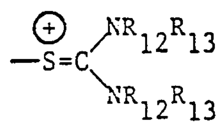 oder 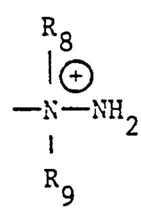

bedeuten, wobei in obigen Formeln q für 0 oder 1, $R_8$ und $R_9$ unabhängig voneinander für gegebenenfalls substituiertes Alkyl mit 1 bis 6 C-Atomen, $R_{10}$ für gegebenenfalls substituiertes Alkyl mit 1 bis 6 C-Atomen, Cycloalkyl mit 3 bis 7 C-Atomen oder die Gruppe $NR_{12}R_{13}$, $R_{11}$ für Alkyl, $R_{12}$ und $R_{13}$ unabhängig voneinander für Wasserstoff oder gegebenenfalls substituiertes Alkyl, $R_{14}$ und $R_{15}$ unabhängig voneinander für einen gegebenenfalls substituierten Alkyl- oder Aralkylrest, r für eine ganze Zahl von 1 bis 6, $A_1$ für die Ergänzung zu einem aromatischen 5- bis 7-gliedrigen Stickstoffheterocyclus, der gegebenenfalls noch ein oder zwei weitere Stickstoffatome als Ringglieder enthalten kann und der gegebenenfalls verschiedene Substituenten tragen kann, und B für die Ergänzung zu einem gesättigten 5- bis 7-gliedrigen Stickstoffheterocyclus, der gegebenenfalls noch 1 bis 2 Stickstoff-, Sauerstoff- und/oder Schwefelatome als Ringglieder enthalten kann und der gegebenenfalls verschiedene weitere Substituenten tragen kann, stehen.

10. Phthalocyaninverbindungen nach Anspruch 9, worin A eine Gruppe $-SO_2NHR'_7-Y'_2{}^{\oplus}$ bedeutet, worin $R'_7$ für Alkylen mit 2 bis 6 C-Atomen und $Y'_2{}^{\oplus}$ für eine Gruppe der Formel

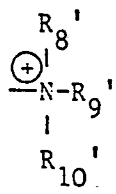 , 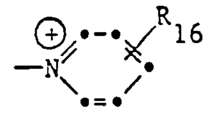 , 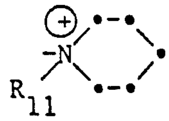 oder 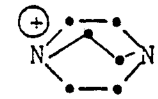

stehen, worin $R_8'$ und $R_9'$ unabhängig voneinander für unsubstituiertes oder durch Hydroxy, Cyano, Halogen oder Phenyl substituiertes Alkyl mit 1 bis 4 C-Atomen stehen, $R_{10}$ die Bedeutungsmöglichkeiten von $R_8$ hat und zusätzlich für Cyclohexyl oder die Aminogruppe stehen kann, und $R_{11}$ für $C_1$-$C_4$-Alkyl und $R_{16}$ für $C_1$-$C_4$-Alkyl, $C_1$-$C_4$-Alkoxy, Halogen, Carboxy, Carbalkoxy oder Hydroxy stehen.

11. Phthalocyaninverbindungen nach Anspruch 1, worin A eine nichtionische wasserlöslichmachende Gruppe und Z 0 ist.

12. Phthalocyaninverbindungen nach Anspruch 11, worin A für eine Gruppe der Formel

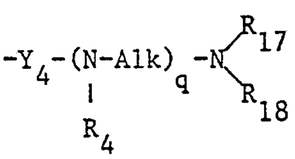 , 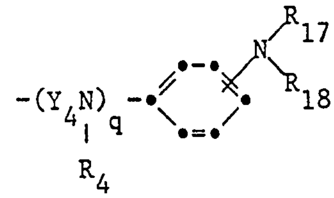 , 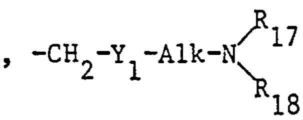 ,

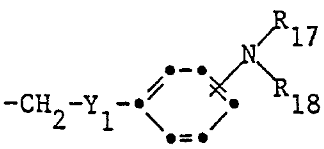 oder $-(CH_2CH_2O)_gH$

steht, worin $Y_1$ S oder $NR_4$, $Y_4$ $-SO_2-$ oder -CO-, vorzugsweise $-SO_2-$, Alk gegebenenfalls substituiertes Alkylen, $R_{17}$ und $R_{18}$ unabhängig voneinander Wasserstoff, Alkyl, Hydroxyalkyl, Cyanoalkyl, Sulfoalkyl, Carboxyalkyl oder Halogenalkyl, unsubstituiertes oder mit Halogen, Alkyl oder Alkoxy, Sulfo oder Carboxy substituiertes Phenyl oder $R_{17}$ und $R_{18}$ zusammen mit dem Stickstoffatom, an das sie gebunden sind, einen gesättigten 5- oder 6-gliedrigen heterocyclischen Ring, der zusätzlich noch ein Stickstoff- oder Sauerstoffatom als Ringglied enthalten kann, $R_4$ Wasserstoff oder gegebenenfalls substituiertes Alkyl, q 0 oder 1 und g eine Zahl von 4 bis 50 bedeuten.

13. Phthalocyaninverbindungen nach Anspruch 12, worin A für eine Gruppe der Formel

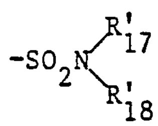 , 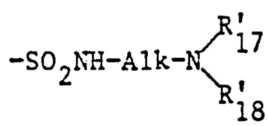 oder $-(CH_2CH_2O)_{g'}H$

steht, worin Alk $C_2$-$C_6$-Alkylen, $R'_{17}$ und $R'_{18}$ unabhängig voneinander Wasserstoff, Alkyl, Hydroxyalkyl, Cyanoalkyl oder Halogenalkyl mit jeweils 1 bis 6 C-Atomen, Phenyl oder zusammen mit dem Stickstoffatom, an das sie gebunden sind, den Piperidin-, Piperazin- oder Morpholinring und g' eine Zahl von 8 bis 40 bedeuten.

14. Phthalocyaninverbindungen nach Anspruch 1, die im Molekül nur eine Art von wasserlöslichmachenden Gruppen (anionisch, kationisch oder nichtionisch) enthalten.

15. Phthalocyaninverbindungen nach Anspruch 1, die im Molekül 2 oder 3 Arten von wasserlöslichmachenden Gruppen (anionische, kationische und/oder nichtionische) enthalten.

16. Phthalocyaninverbindungen nach einem der Ansprüche 1-15, worin Y Halogen, Cyano, Nitro, gegebenenfalls substituiertes Alkyl oder gegebenenfalls substituiertes Phenyl bedeutet.

17. Phthalocyaninverbindungen nach Anspruch 16, worin Y Halogen, Alkyl, Phenyl, Halogenphenyl, Alkylphenyl oder Alkoxyphenyl bedeutet.

18. Phthalocyaninverbindungen nach Anspruch 17, worin Y Chlor, Brom, $C_1$-$C_4$-Alkyl oder Phenyl, vorzugsweise Chlor oder Brom, bedeutet.

19. Phthalocyaninverbindungen nach einem der Ansprüche 1 bis 18, worin n eine beliebige Zahl zwischen 1 und 4 und p eine beliebige Zahl zwischen 0 und 4 bedeuten.

20. Phthalocyaninverbindungen nach Anspruch 1, worin A eine Gruppe der Formel

$-SO_3^{\ominus}$, $-SO_2^{\ominus}$, $-SO_2\overset{\ominus}{N}-CN$, $-SO_2\overset{\ominus}{N}-SO_2-R_6'$ , $-SO_2NH-R_7''-SO_3^{\ominus}$ ,

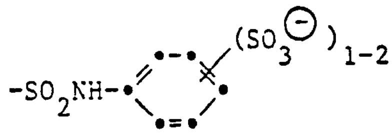 , 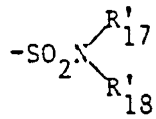 , 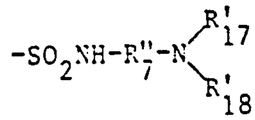 ,

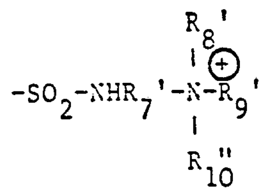 oder 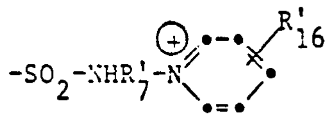

Z als Gegenion für anionische Gruppen ein Wasserstoff-, Alkalimetall-, unsubstituiertes oder substituiertes Ammoniumion, für kationische Gruppen ein Halogenid-, Alkylsulfat-, gegebenenfalls substituiertes Phenylsulfonat, Sulfat- oder Phosphation oder das Ion einer organischen Carbonsäure und für nichtionische Gruppen 0, n eine beliebige Zahl von 1 bis 4, p eine beliebige Zahl von 0 bis 4 und Y Halogen, Alkyl oder Phenyl bedeuten, wobei in obigen Formeln $R'_6$ $C_1$-$C_4$-Alkyl, Phenyl, ($C_1$-$C_4$-Alkyl)phenyl, Chlorphenyl oder Methoxyphenyl, $R'_7$ $C_2$-$C_6$-Alkylen, $R''_7$ $C_1$-$C_6$-Alkylen, $R'_8$ $R'_9$ und $R''_{10}$ unabhängig voneinander unsubstituiertes oder durch Hydroxy, Halogen oder Phenyl substituiertes $C_1$-$C_4$-Alkyl, $R'_{16}$ $C_1$-$C_4$-Alkyl, Halogen oder Hydroxy und $R'_{17}$, und $R'_{18}$ unabhängig voneinander Wasserstoff, Alkyl, Hydroxyalkyl, Halogenalkyl mit jeweils 1-6 C-Atomen, Phenyl oder zusammen mit dem Stickstoffatom, an das sie gebunden sind, den Piperidin-, Piperazin- oder Morpholinring bedeuten, wobei im Molekül verschiedene Arten von Substituenten A oder/und Y vorhanden sein können.

21. Phthalocyaninverbindungen nach Anspruch 20, worin A eine Gruppe der Formel $SO_3^{\ominus}$, $-SO_2^{\ominus}N-CN$ oder $-SO_2^{\ominus}N-SO_2-R'_6$ ist, worin $R'_6$ wie in Anspruch 25 definiert ist und Z ein Wasserstoff-, Alkalimetall- oder Ammoniumion bedeutet.

22. Phthalocyaninverbindungen nach Anspruch 21, worin A $SO_3^{\ominus}$, Z ein Wasserstoff-, Alkalimetall- oder Ammoniumion, n eine beliebige Zahl von 1 bis 4, vorzugsweise 2 bis 4, und p eine beliebige Zahl von 0 bis 4, vorzugsweise von 0 bis 2, bedeuten.

23. Verwendung der in Anspruch 1-22 definierten Phthalocyaninverbindungen als Photobleichmittel zum Bleichen von bzw. Fleckenentfernen aus Textilien und zum Bekämpfen von Mikroorganismen in oder auf organischen oder anorganischen Substraten bzw. zum Schützen derselben vor dem Befall durch Mikroorganismen.

24. Photosensibilisierendes (photoaktivierendes) oder/und Singlett-Sauerstoff produzierendes Mittel, das eine oder mehrere in den Ansprüchen 1-22 definierte Phthalocyaninverbindung(en) enthält.

**Claims**

1. A phthalocyanine compound of the formula

$$\left[\mathrm{MePc}\right]\begin{matrix}(AZ)_n \\ (Y)_p\end{matrix}$$

in which MePc is the phthalocyanine or naphthalocyanine ring system

A is an anionic, nonionic or cationic group which imparts solubility in water,

Z is the counter-ion to A, if A is an ionic group, and

Z is zero if A is a nonionic group,

n is any desired number from 1 to 8,

Y is a neutral substituent which does not impart solubility in water

and

p is any desired number from 0 to 6, it being possible for the groups AZ and Y present in the molecule to be

identical or different in either case, and the sum of n + p being not more than 8.

2. A phthalocyanine compound according to Claim 1, in which A is an anionic group and Z is a cation.

3. A phthalocyanine compound according to Claim 2, in which A is a sulfo, carboxyl, phosphate, sulfate, sulfinyl, disulfimide or cyanimide group or a radical containing one or more of the abovementioned groups.

4. A phthalocyanine compound according to Claim 3, in which A is one of the following groups:

$-SO_3^{\ominus}$, $-COO^{\ominus}$, $-SO_2^{\ominus}$, $-SO_2-Alk-OSO_3^{\ominus}$, $-SO_2-Alk-SO_3^{\ominus}$,

$-Y_4-N(R_4)-Alk-OSO_3^{\ominus}$, $-Y_4-N(R_4)-Alk-SO_3^{\ominus}$, $-(CH_2CH_2O)_m-SO_3^{\ominus}$,

$-(CH_2CH_2O)_m-COO^{\ominus}$, 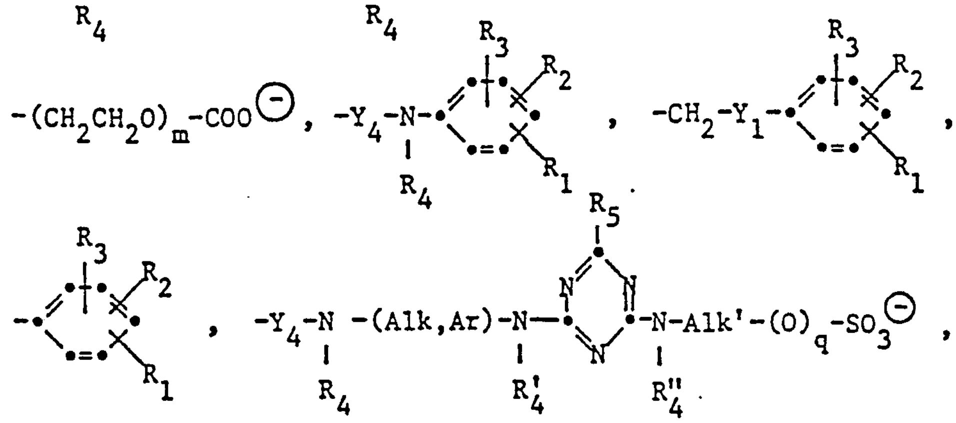

-$SO_2^{\ominus}N$-CN or -$SO_2^{\ominus}N$-$SO_2$-$R_6$ in which $R_1$, $R_2$ and $R_3$ independently of one another are hydrogen, alkyl, hydroxyl, $SO_3^{\ominus}$ or -$COO^{\ominus}$, at least one of these substituents being $SO_3^{\ominus}$ or $COO^{\ominus}$, $R_4$, $R'_4$ and $R''_4$ independently of one another are substituted or unsubstituted alkyl or hydrogen, $R_5$ is hydrogen or halogen, $R_6$ is substituted or unsubstituted alkyl or substituted or unsubstituted phenyl or naphthyl, Ar is a substituted or unsubstituted phenyl or naphthyl radical, Alk and Alk' are each a substituted or unsubstituted alkylene group, $Y_1$ is -N-$R_4$ or S, $Y_4$ is -$SO_2$- or -CO-, preferably -$SO_2$-, m is a number from 1 to 30 and q is or 1.

5. A phthalocyanine compound according to Claim 4, in which A is a group of the formula $SO_3^{\ominus}$, -$COO^{\ominus}$, -$SO_2^{\ominus}$, -$SO_2^{\ominus}N$-CN or -$SO_2^{\ominus}N$-$SO_2$-$R_6$ in which $R_6$ is $C_1$-$C_4$alkyl, phenyl, ($C_1$-C4alkyl)-phenyl, chlorophenyl or methoxyphenyl.

6. A phthalocyanine compound according to Claim 5, in which A is $SO_3^{\ominus}$ and Z is a hydrogen ion, alkali metal ion or unsubstituted or substituted ammonium ion.

7. A phthalocyanine compound according to Claim 1, in which A is a cationic group and Z is an anion.

8. A phthalocyanine compound according to Claim 7, in which A is a quaternary ammonium group or a ternary sulfonium group or a radical containing one or more of the abovementioned groups.

9. A phthalocyanine compound according to Claim 7, in which A is a group of the formula

$-SO_2N(R_4)-R_7-Y_2^{\oplus}$, $-CON(R_4)-R_7-Y_2^{\oplus}$, $-(CH_2)_q-Y_3^{\oplus}$ or $-CH_2NHCOCH_2-Y_3^{\oplus}$

in which q is 0 or 1, $R_7$ is unbranched or branched alkylene having 1 to 8 C atoms or 1,3-phenylene or 1,4-phenylene, $R_4$ is hydrogen or substituted or unsubstituted alkyl, $Y_2^{\oplus}$ is a group of the formula

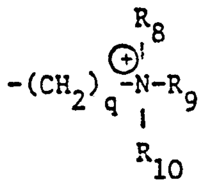, 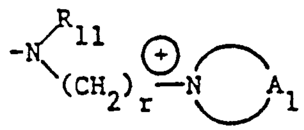, 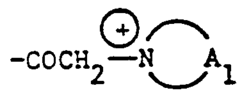,

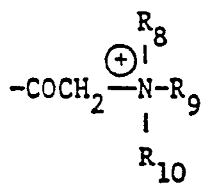

or in the event that $R_7$ = alkylene, $Y_2^{\oplus}$ is also a group of the formula

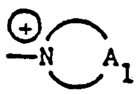, 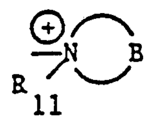, 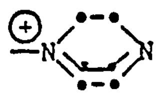, 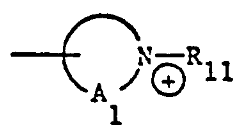,

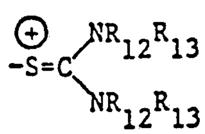 oder 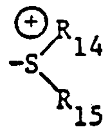,

and $Y_3^{\oplus}$ a group of the formula

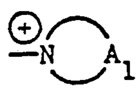, 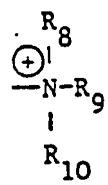, 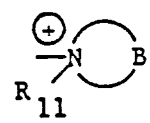,

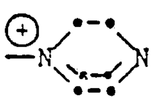, 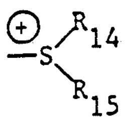, 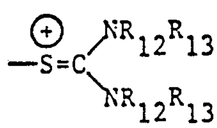 or 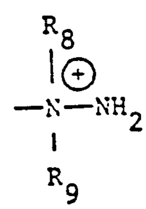

q in the above formulae being 0 or 1, $R_8$ and $R_9$ independently of one another being substituted or unsubstituted alkyl having 1 to 6 C atoms, $R_{10}$ being substituted or unsubstituted alkyl having 1 to 6 C atoms, cycloalkyl having 3 to 7 C atoms or the group $NR_{12}R_{13}$, $R_{11}$ being alkyl, $R_{12}$ and $R_{13}$ independently of one another being hydrogen or substituted or unsubstituted alkyl, $R_{14}$ and $R_{15}$ independently of one another being a substituted or unsubstituted alkyl or aralkyl radical, r being an integer from 1 to 6, $A_1$ being the groups needed to complete an aromatic, 5-mem-bered to 7-membered nitrogen heterocyclic structure which can, if desired, also contain 1 or 2 further nitrogen atoms as ring members and which can, if desired, carry different substituents, and B being the groups needed to complete a saturated, 5-membered to 7-membered nitrogen heterocyclic structure which can, if desired, also contain 1 to 2 nitrogen, oxygen and/or sulfur atoms as ring members and which can, if desired, carry different further substituents.

10. A phthalocyanine compound according to Claim 9, in which A is a group $-SO_2NHR_7-Y_2^{\oplus}$ in which $R_7$ is alkylene having 2 to 6 C atoms and $Y_2^{\oplus}$ is a group of the formula

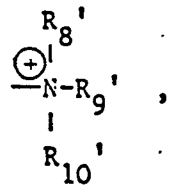, 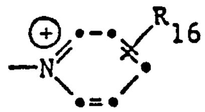, 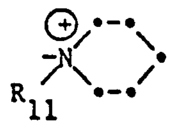 or 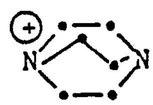

in which $R_8$ and $R_9$ independently of one another are alkyl which has 1 to 4 C atoms and is unsubstituted or substituted by hydroxyl, cyano, halogen or phenyl, $R_{10}$ has the possible meanings of $R_8$ and can additionally be cyclohexyl or the amino group, and $R_{11}$ is $C_1$-$C_4$alkyl and $R_{16}$ is $C_1$-$C_4$alkyl, $C_1$-$C_4$alkoxy, halogen, carboxyl, carboalkoxy or hydroxyl.

11. A phthalocyanine compound according to Claim 1, in which A is a nonionic group which imparts solubility in water and Z is 0.

12. A phthalocyanine compound according to Claim 11, in which A is a group of the formula

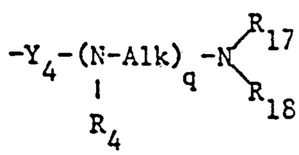, 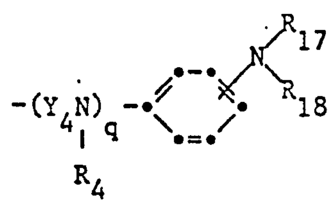, 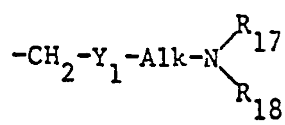,

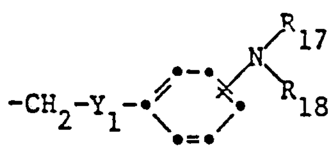 or $-(CH_2CH_2O)_gH$

in which $Y_1$ is S or $NR_4$, $Y_4$ is $-SO_2-$ or -CO-, preferably $-SO_2-$, Alk is substituted or unsubstituted alkylene, $R_{17}$ and $R_{18}$ independently of one another are hydrogen, alkyl, hydroxyalkyl, cyanoalkyl, sulfoalkyl, carboxyalkyl or halogenoalkyl, phenyl which is unsubstituted or substituted by halogen, alkyl or alkoxy, sulfo or carboxy, or $R_{17}$ and $R_{18}$, together with the nitrogen atom to which they are attached, are a saturated, 5-membered or 6-membered heterocyclic ring which can, in addition, also contain a nitrogen or oxygen atom as a member of the ring, $R_4$ is hydrogen or substituted or unsubstituted alkyl, q is 0 or 1 and g is a number from 4 to 50.

13. A phthalocyanine compound according to Claim 12, in which A is a group of the formula

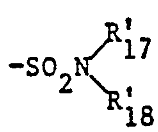, 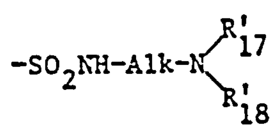 or $-(CH_2CH_2O)_{g'}H$

in which Alk is $C_2$-$C_6$alkylene, $R_{17}$ and $R_{18}$ independently of one another are hydrogen, alkyl, hydroxyalkyl, cyanoalkyl or halogenoalkyl having in each case 1 to 6 C atoms, or phenyl or, together with the nitrogen atom to which they are attached, are the piperidine, piperazine or morpholine ring and g' is a number from 8 to 40.

14. A phthalocyanine compound according to Claim 1, the molecule of which contains only one type of groups imparting solubility in water (anionic, cationic or nonionic).

15. A phthalocyanine compound according to Claim 1, the molecule of which contains 2 or 3 types of groups imparting solubility in water (anionic, cationic and/or nonionic).

16. A phthalocyanine compound according to any one of Claims 1-15, in which Y is halogen, cyano, nitro, substituted or unsubstituted alkyl or substituted or unsubstituted phenyl.

17. A phthalocyanine compound according to Claim 16, in which Y is halogen, alkyl, phenyl, halogenophenyl, alkylphenyl or alkoxyphenyl.

18. A phthalocyanine compound according to Claim 17, in which Y is chlorine, bromine, $C_1$-$C_4$alkyl or phenyl, preferably chlorine or bromine.

19. A phthalocyanine compound according to any one of Claims 1 to 18, in which n is any desired number between 1 and 4 and p is any desired number between 0 and 4.

20. A phthalocyanine compound according to Claim 1, in which A is a group of the formula

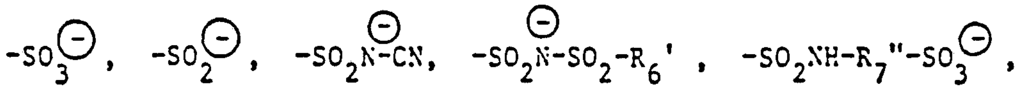

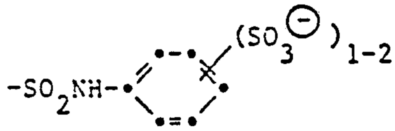 , 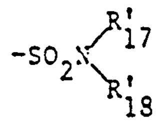 , 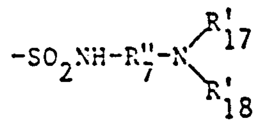 ,

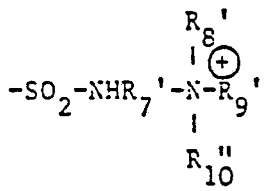 or 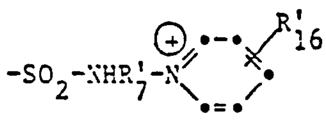

Z, as a counter-ion for anionic groups, is a hydrogen ion, alkali metal ion or unsubstituted or substituted ammonium ion, for cationic groups is a halide ion, alkylsulfate ion, a substituted or unsubstituted phenylsulfonate ion, a sulfate ion or a phosphate ion or the ion of an organic carboxylic acid, and, for nonionic groups, is 0, n is any desired number from 1 to 4, p is any desired number from 0 to 4 and Y is halogen, alkyl or phenyl, $R_6$ in the above formulae being $C_1$-$C_4$alkyl, phenyl, ($C_1$-$C_4$alkyl)-phenyl, chlorophenyl or methoxyphenyl, $R_7$ being $C_2$-$C_6$alkylene, $R_7$ being $C_1$-$C_6$alkylene, $R_8$, $R_9$ and $R_{10}$ independently of one another being $C_1$-$C_4$alkyl which is unsubstituted or substituted by hydroxyl, halogen or phenyl, $R_{16}$ being $C_1$-$C_4$alkyl, halogen or hydroxyl and $R_{17}$ and $R_{18}$ independently of one another being hydrogen, alkyl, hydroxyalkyl or halogenoalkyl being 1-6 C atoms in each case, or phenyl or, together with the nitrogen atom to which they are attached, being the piperidine, piperazine or morpholine ring, it being possible for different types of substituents A and/or Y to be present in the molecule.

21. A phthalocyanine compound according to Claim 20, in which A is a group of the formula $SO_3^{\ominus}$, $-SO_2N^{\ominus}$-CN or $-SO_2^{\ominus}N-SO_2-R_6$ in which $R_6$ is as defined in Claim 20 and Z is a hydrogen, alkali metal or ammonium ion.

22. A phthalocyanine compound according to Claim 21, in which A is $SO_3^{\ominus}$, Z is a hydrogen, alkali metal or ammonium ion, n is any desired number from 1 to 4, preferably 2 to 4, and p is any desired number from 0 to 4, preferably from 0 to 2.

23. The use of the phthalocyanine compounds defined in Claims 1-22 as photobleaching agents for bleaching or removing spots from textiles and for controlling microorganisms in or on organic or inorganic substrates or for protecting the latter against attack by microorganisms.

24. A photosensitising (photoactivating) agent or an agent producing singlet oxygen, which contains one or more phthalocyanine compound(s) defined in Claims 1-22.

**Revendications**

1. Composés phtalocyanines de formule

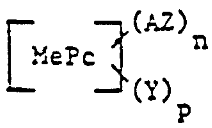

dans laquelle

MePc représente le système cyclique phtalocyanine ou naphtalocyanine de Ge(IV),

A est un groupe hydrosolubilisasnt, anionique, nonionique ou cationique,

Z est le contre-ion de A, si A est un groupe ionique,

et

Z n'est rien si A est un groupe non-ionique,

n est un nombre quelconque de 1 à 8,

Y est un substituant neutre non-hydrosolubilisant, et

p est un nombre quelconque de 0 à 6, les groupes AZ et

Y se trouvant dans la molécule pouvant cependant être identiques ou différents, et la somme n + p étant au plus égale à 8.

2. Composés phtalocyanines selon la revendication 1, dans lesquels A est un groupe anionique et Z est un cation.

3. Composés phtalocyanines selon la revendication 2, dans lesquels A est un groupe sulfo, carboxyle, phosphate, sulfate, sulfinyle, disulfimide ou cyanimide, ou un radical contenant un ou plusieurs des groupes mentionnés ci-dessus.

4. Composés phtalocyanines selon la revendication 3, dans lesquels A est l'un des groupes suivants :

$-SO_3^{\ominus}$, $-COO^{\ominus}$, $-SO_2^{\ominus}$, $-SO_2-Alk-OSO_3^{\ominus}$, $-SO_2-Alk-SO_3^{\ominus}$,

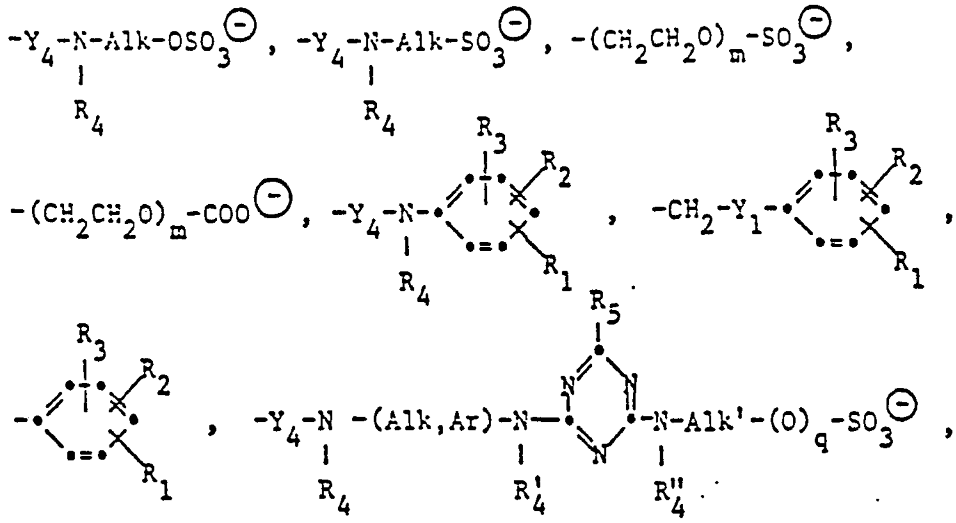

$-(CH_2CH_2O)_m-SO_3^{\ominus}$, $-(CH_2CH_2O)_m-COO^{\ominus}$,

$-SO_2\overset{\ominus}{N}-CN$ ou $-SO_2\overset{\ominus}{N}-SO_2-R_6$,

où $R_1$, $R_2$ et $R_3$, indépendamment les uns des autres, représentent chacun un hydrogène, un radical alkyle, ou le radical hydroxy, $SO_3^{\ominus}$ ou $COO^{\ominus}$, au moins l'un de ces substituants étant $SO_3^{\ominus}$ ou $COO^{\ominus}$, $R_4$, $R'_4$ et $R''_4$, indépendamment les uns des autres, sont chacun un radical alkyle éventuellement substitué ou un hydrogène, $R_5$ est un hydrogène ou un halogène, $R_6$ est un radical alkyle éventuellement substitué, ou phényle ou naphtyle éventuellement substitué, Ar est un radical phényle ou naphtyle éventuellement substitué, Alk et Alk' représentent chacun un groupe alkylène éventuellement substitué, $Y_1$ est $N-R_4$ ou S, $Y_4$ est $-SO_2-$ ou $-CO-$, de préférence $-SO_2-$, m est un nombre de 1 à 30, et q vaut 0 ou 1.

5. Composés phtalocyanines selon la revendication 4, dans lesquels A est un groupe de formule $-SO_3^{\ominus}$, $-COO^{\ominus}$, $-SO_2^{\ominus}$, $-SO_2^{\ominus}N-CN$ ou $-SO_2^{\ominus}N-SO_2-R'_6$, $R'_6$ représentant un radical alkyle en $C_1$-$C_4$, le radical phényle, un radical (alkyle en $C_1$-$C_4$)phényle, ou le radical chlorophényle ou méthoxyphényle.

6. Composés phtalocyanines selon la revendication 5, dans lesquels A est $SO_3^{\ominus}$, et Z est un hydrogène ou un ion de métal alcalin ou un ion ammonium, substitué ou non.

7. Composés phtalocyanines selon la revendication 1, dans lesquels A est un groupe cationique et Z est un anion.

8. Composés phtalocyanines selon la revendication 7, dans lesquels A est un groupe ammonium quaternaire ou un groupe sulfonium ternaire, ou encore un radical contenant un ou plusieurs des groupes mentionnés ci-dessus.

9. Composés phtalocyanines selon la revendication 7, dans lesquels A représente un groupe de formule

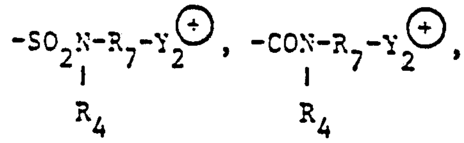, $-(CH_2)_q-Y_3^{\oplus}$ ou $-CH_2NHCOCH_2-Y_3^{\oplus}$

où q vaut 0 ou 1, $R_7$ est un radical alkylène ramifié ou nonramifié ayant de 1 à 8 atomes de carbone, ou le radical 1,3- ou 1,4-phénylène, $R_4$ est un hydrogène ou un radical alkyle éventuellement substitué, $Y_2^{\oplus}$ est un groupe de formule

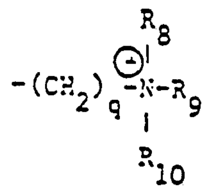 , 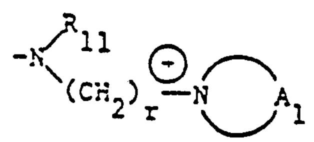 , 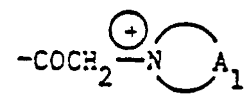 ,

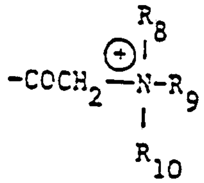

et, si $R_7$ est un radical alkylène, peut aussi représenter un groupe de formule

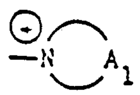 , 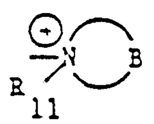 , 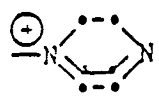 , 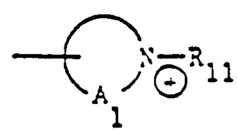 ,

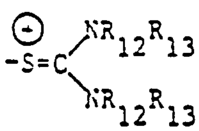 ou 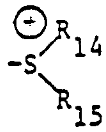 ,

$Y_3^{\oplus}$ est un groupe de formule FIGUR

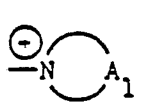 , 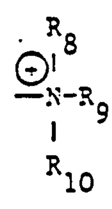 , 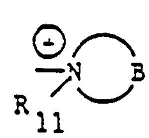 ,

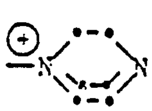 , 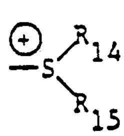 , 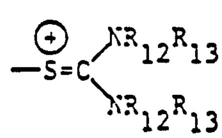 ou 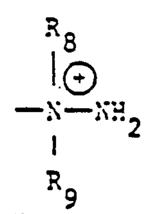

et, dans les formules ci-dessus, q vaut 0 ou 1, $R_8$ et $R_9$, indépendamment l'un de l'autre, représentent chacun un radical alkyle éventuellement substitué ayant de 1 à 6 atomes de carbone, $R_{10}$ est un radical alkyle éventuellement substitué ayant de 1 à 6 atomes de carbone, un radical cycloalkyle ayant de 3 à 7 atomes de carbone ou le groupe $NR_{12}R_{13}$, $R_{11}$ est un radical alkyle, $R_{12}$ et $R_{13}$, indépendamment l'un de l'autre, représentent chacun un hydrogène ou un radical alkyle éventuellement substitué, $R_{14}$ et $R_{15}$, indépendamment l'un de l'autre, représentent chacun un radical alkyle ou aralkyle éventuellement substitué, r est un nombre entier de 1 à 6, $A_1$ est le complément permettant de former un hétérocycle azoté aromatique de 5 à 7 chaînons,

lequel peut éventuellement encore comporter à titre de chaînons un ou deux atomes d'azote supplémentaires et peut éventuellement porter différents substituants, et B représente le complément permettant de former un hétérocycle azoté saturé de 5 à 7 chaînons, lequel peut éventuellement encore contenir en tant que chaînons 1 à 2 atomes d'azote, d'oxygène et/ou de soufre et éventuellement peut porter différents autres substituants.

10. Composés phtalocyanines selon la revendication 9, dans lesquels A désigne un groupe $-SO_2NHR'_7-Y'_2{}^{\oplus}$, où $R'_7$ représente un radical alkylène ayant de 2 à 6 atomes de carbone et $Y'_2{}^{\oplus}$ est un groupe de formule

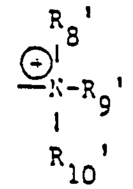 , 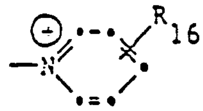 , 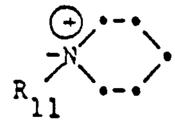 ou 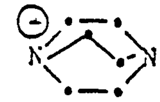

dans laquelle $R'_8$ et $R'_9$, indépendamment l'un de l'autre, désignent chacun un radical alkyle ayant de 1 à 4 atomes de carbone, non substitué ou substitué par un radical hydroxy, cyano, halogène ou phényle, $R_{10}$ a les significations possibles de $R_8$ et, de plus, peut représenter le groupe cyclohexyle ou le groupe amino, et $R_{11}$ désigne un radical alkyle en $C_1$-$C_4$ et $R_{16}$ un radical alkyle en $C_1$-$C_4$, alcoxy en $C_1$-$C_4$, un halogène, le radical carboxy, un radical carbalcoxy ou le radical hydroxy.

11. Composés phtalocyanines selon la revendication 1, dans lesquels A désigne un groupe non-ionique hydrosolubilisant, et Z vaut 0.

12. Composés phtalocyanines selon la revendication 11, dans lesquels A désigne un groupe de formule

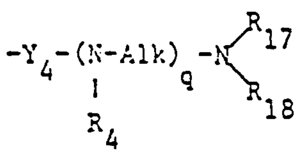 , 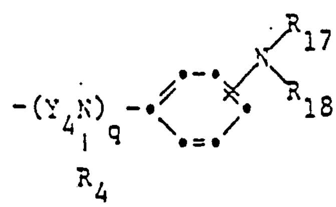 , 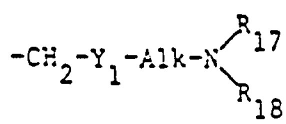 ,

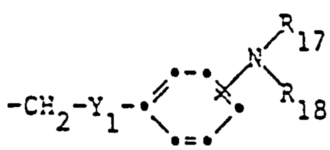 ou $-(CH_2CH_2O)_gH$

dans laquelle $Y_1$ est S ou $NR_4$, $Y_4$ est $-SO_2-$ ou -CO-, de préférence $-SO_2-$, Alk désigne un radical alkylène éventuellement substitué, $R_{17}$ et $R_{18}$, indépendamment l'un de l'autre, sont chacun un hydrogène, un radical alkyle, hydroxyalkyle, cyanoalkyle, sulfoalkyle, carboxyalkyle ou halogènalkyle, un radical phényle non-substitué ou substitué par un halogène, un radical alkyle ou alcoxy ou le radical sulfo ou carboxy, ou bien $R_{17}$ et $R_{18}$, pris avec l'atome d'azote auquel ils sont liés, forment un noyau hétérocyclique saturé à 5 ou 6 chaînons, lequel peut en outre contenir encore un atome d'azote ou d'oxygène en tant que chaînon, $R_4$ est un hydrogène ou un radical alkyle éventuellement substitué, q vaut 0 ou 1, et g est un nombre de 4 à 50.

13. Composés phtalocyanines selon la revendication 12, dans lesquels A désigne un groupe de formule

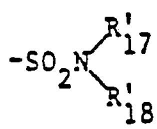 , 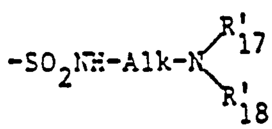 ou $-(CH_2CH_2O)_{g'}H$

dans laquelle Alk représente un radical alkylène en $C_2$-$C_6$, $R'_{17}$ et $R'_{18}$, indépendamment l'un de l'autre, représentent chacun un hydrogène, un radical alkyle, hydroxyalkyle, cyanoalkyle ou halogènalkyle ayant chacun de 1 à 6 atomes de carbone, ou le radical phényle, ou encore, avec l'atome d'azote auquel ils sont liés, forment le noyau pipéridine, pipérazine ou morpholine, et g' est un nombre de 8 à 40.

14. Composés phtalocyanines selon la revendication 1, qui, dans leur molécule, ne contiennent qu'un type de groupes hydrosolubilisants (anionique, cationique ou nonionique).

15. Composés phtalocyanines selon la revendication 1, qui contiennent dans leur molécule 2 ou 3 types de groupes hydrosolubilisants(anioniques, cationiques et/ou nonioniques).

16. Composés phtalocyanines selon l'une des revendications 1 à 15, dans lesquels Y désigne un radical

halogéno, cyano, nitro, alkyle éventuellement substitué ou phényle éventuellement substitué.

17. Composés phtalocyanines selon la revendication 16, dans lesquels Y désigne un radical halogéno, alkyle, phényle, halogénophényle, alkylphényle ou alcoxyphényle.

18. Composés phtalocyanines selon la revendication 17, dans lesquels Y désigne un radical chloro, bromo, alkyle en $C_1$-$C_4$ ou phényle, de préférence chloro ou bromo.

19. Composés phtalocyanines selon l'une des revendications 1 à 18, dans lesquels n est un nombre quelconque compris entre 1 et 4, et p est un nombre quelconque compris entre 0 et 4.

20. Composés phtalocyanines selon la revendication 1, dans lesquels A est un groupe de formule

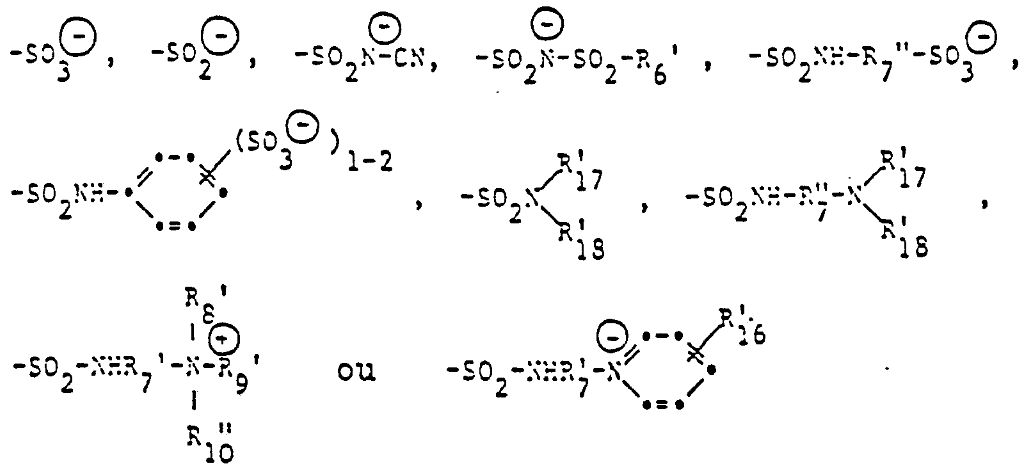

Z, en tant que contre-ion de groupes anioniques, est un ion hydrogène, métal alcalin, ammonium substitué ou non-substitué, et, en tant que contre-ion de groupes cationiques, est un ion halogénure, alkylsulfate, phénylsulfonate éventuellement substitué, sulfate ou phosphate, ou encore est l'ion d'un acide carboxylique organique et, en tant que contre-ion de groupes non-ioniques, n'est rien, n est un nombre quelconque de 1 à 4, p est un nombre quelconque de 0 à 4, et Y est un radical halogéno, alkyle ou phényle, et, dans les formules ci-dessus, $R'_6$ est un radical alkyle en $C_1$-$C_4$, phényle, (alkyle en $C_1$-$C_4$)phényle, chlorophényle ou méthoxyphényle, $R'_7$ est un radical alkylène en $C_2$-$C_6$, $R''_7$ est un radical alkylène en $C_1$-$C_6$, $R'_8$, $R'_9$ et $R''_{10}$, indépendamment les uns des autres, représentent chacun un radical alkyle en $C_1$-$C_4$, non-substitué ou substitué par un radical hydroxy, halogéno ou phényle, $R'_{16}$ est un radical alkyle en $C_1$-$C_4$, halogéno ou hydroxy, et $R'_{17}$ et $R'_{18}$, indépendamment l'un de l'autre, représentent chacun un hydrogène, un radical alkyle, hydroxyalkyle, halogénalkyle ayant chacun de 1 à 6 atomes de carbone, ou le radical phényle, ou encore, avec l'atome d'azote auquel ils sont liés, forment le noyau pipéridine, pipérazine ou morpholine, différents types de substituants A et/ou Y pouvant être présents dans la molécule.

21. Composés phtalocyanines selon la revendication 20, dans lesquels A est un groupe de formule $SO_3^{\ominus}$, $-SO_2N^{\ominus}-CN$ ou $-SO_2N^{\ominus}-SO_2-R'_6$, où $R'_6$ est défini comme dans la revendication 25, et Z est un ion hydrogène, métal alcalin ou ammonium.

22. Composés phtalocyanines selon la revendication 2l, dans lesquels A est $SO_3^{\ominus}$, Z est un ion hydrogène, métal alcalin ou ammonium, n est un nombre quelconque de 1 à 4, de préférence de 2 à 4, et p est un nombre quelconque de 0 à 4, de préférence de 0 à 2.

23. Utilisation des composés phtalocyanines définis dans les revendications 1 à 22 en tant qu'agents photoblanchissants destinés au blanchiment ou au détachage de textiles, et à la lutte contre des microorganismes dans ou sur des substrats organiques ou inorganiques, ou encore à la protection de ces derniers contre une infestation par des microorganismes.

24. Agent photosensibilisateur (photoactivateur) et/ou produisant de l'oxygène sous forme singulet, qui contient un ou plusieurs des composés phtalocyanines définis dans les revendications 1 à 22.